# EUROPEAN PATENT SPECIFICATION

(11) **EP 3 843 796 B1**
(45) Date of publication and mention of the grant of the patent: **20.04.2022**
(21) Application number: 19790784.3
(22) Date of filing: 27.08.2019
(51) Int. Cl.: A61L 15/24, A61L 15/28, A61L 15/42

(54) **COMPOSITE DRESSINGS, MANUFACTURING METHODS AND APPLICATIONS THEREOF**
ZUSAMMENGESETZTE WUNDAUFLAGEN, HERSTELLUNGSVERFAHREN UND ANWENDUNGEN DAVON
PANSEMENTS COMPOSITES, LEURS PROCÉDÉS DE FABRICATION ET LEURS APPLICATIONS

(30) Priority: 27.08.2018 IN 201841032029
(43) Date of publication of application: 07.07.2021
(73) Proprietor: ADVAMEDICA INC., Cambridge, MA 02139 (US)
(72) Inventor: MAVELY, Leo, Ahmedabad 380054 (IN); SONAJE, Kiran, Ahmedabad 380054 (IN); AGRAWAL, Animesh, Ahmedabad 380054 (IN)
(74) Representative: Nederlandsch Octrooibureau
(86) International application number: PCT/IB2019/057215
(87) International publication number: WO 2020/044237

(56) References cited:
- EP-A2- 1 430 911
- CN-B- 104 189 945

## Description

### TECHNICAL FIELD

The present disclosure broadly relates to the fields of medical dressings and wound management. Particularly, the disclosure provides a biomaterial based composite dressing and a method for preparation thereof, which enables production of said composite dressing without use of an adhesive between its distinct layers. More particularly, the disclosure provides a composite dressing comprising two or more physically distinct components, at least one of which is made of biomaterials, having applications such as hemostatic dressings for deep cavity wounds, puncture wounds, post-partum hemorrhage as well as internal hemostatic dressings. Said composite dressing comprises no adhesive, stitching or chemical crosslinking agent between multiple layers forming the dressing.

### BACKGROUND OF THE DISCLOSURE

Although several absorbable hemostats and sealants are commercially available, they are not widely used due to higher costs, limited absorption capacity, and their inability to control profuse and/or consistent bleeding. Therefore, laparotomy sponges and gauze swabs are routinely used during surgical procedures to absorb fluids and to "wall off' the surgical site. However, such conventional gauze dressings do not promote hemostasis which leads to longer surgeries and time in operation theaters eventually leading to higher costs of surgeries to patients. Similar is the case with conventional dressings that are available for deep cavity wounds, puncture wounds, post-partum hemorrhage, as well as internal bleedings. Therefore, advanced hemostatic dressings are desired which offer high fluid absorbency at the same time promote the hemostasis.

Composite dressings are wound covers that combine physically distinct components such as a porous matrix and fabric support into a single product to provide multiple functions such as high fluid absorption, superior dry and wet strength, bacterial barrier, flexibility and ease of handling and adhesion. Conventionally, composite dressings are comprised of multiple layers that are bonded together by an adhesive layer or stitching.

The adhesives, however, are generally not suitable for in situ applications and are not supposed to come in direct contact with the wound surface. Further, the adhesives used in such dressings often lose their functionality after absorbing fluids, and the swellable porous layers tend to get detached or break apart from the backing layer when the adhesives or backing layers do not expand in same proportion as the porous matrix. In some cases, absorbent pads are stitched on gauze dressings, however not all materials are suitable for stitching, especially the foams obtained using natural polymers such as cellulose, alginate or chitosan tend to break during stitching and lose their strength. In EP1430911A2 and CN104189945B a fabric is at least partially embedded in a porous matrix.

Methods of prior art involve solvent treatment on foam and fibers, or compression of the multi-layered substrate, which may lead to collapse of porous structure of the matrix and may affect its functionality.

In view of the above drawbacks in the art, composite dressings which do not require adhesives, stitching or chemical crosslinking agents are desirable. Further, it is important that the composite dressings have distinct porous matrix and good mechanical properties without affecting the porous structure of the matrix.

### SUMMARY OF THE DISCLOSURE

Accordingly, the present disclosure relates to a removable composite dressing comprising at least one layer of porous matrix composed of biomaterial and at least one layer of fabric support, wherein the fabric support is at least partially embedded and dissolved in the porous matrix, and wherein at least 50% of the fibers from the fabric support remain undissolved within or outside the porous matrix.

In some embodiments, the porous matrix and the fabric support are both composed of biomaterial, and wherein the porous matrix and the fabric support are both composed of same or different biomaterial.

In further embodiments, the biomaterial is selected from a group comprising chitosan, alginates, starch, cellulose, gelatin, collagen, Polyethylene Glycol (PEG), Polyvinyl Alcohol (PVA), polyurethanes, hyaluronic acid, poly-lysine, poly-glutamic acid, poly(ethylene oxide) (PEO), poly(propylene oxide) (PPO), poly(acrylic acid), poly(methacrylic acid) and their derivatives or any combination thereof. Further, the fabric support is prepared from natural material selected from a group comprising cotton, wool, silk and blends thereof, or synthetic material selected from a group comprising polyamide, polyester, rayon and blends thereof.

In some embodiments, when embedded, thickness of interface between the layer of porous matrix and the layer of fabric support is at least about 1% of the thickness of the fabric support, preferably ranging from about 10% to about 100% of the thickness of the fabric support. The embedding is such that at least 50% of fibers from the fabric support remain undissolved within or outside the porous matrix.

In further embodiments, the composite dressing of the present disclosure has absorption capacity ranging from about 10 times to about 200 times of its original weight. Further, the adhesion force of the composite dressing to application site ranges from about 0.1 MPa to 1 MPa.

The present disclosure further provides a method for preparing the composite dressing as aforementioned, wherein said method comprises:
a) contacting the fabric support with a solution of the biomaterial;
b) at least partially immersing the fabric in the solution of the biomaterial;
c) freezing the solution of the biomaterial comprising the immersed fabric support at a controlled rate; and
d) removing solvent from the frozen biomaterial solution of (c).

In some embodiments, the solution of biomaterial is prepared by dissolving the biomaterial in a solvent selected from a group comprising water, alcohol, DMSO, acid, alkali and organic solvent or any combination thereof.

In other embodiments, the fabric support is obtained by mixing of cross-linked fibers and activated fibers having wetting and swelling properties different from each other.

In some embodiments, wherein the solvent is removed from the frozen biomaterial solution by dipping the frozen biomaterial solution in dehydrating solvent or by carrying out freeze-drying or lyophilization; and wherein the dehydrating solvent is organic solvent selected from a group comprising alcohol, acetone and ethylacetate or any combination thereof.

In some embodiments, the method further comprises saturating the composite dressing with hydration liquid selected from a group comprising water, saline, pro-coagulant material and natural, semisynthetic or synthetic sealants or any combinations thereof, followed by squeezing out excess liquid, before its application to the site of bleeding.

The disclosure also provides for the composite dressing, for use as a medicament.

Further provided is the aforementioned composite dressing for use as medicament in controlling or stopping bleeding, wherein the use comprises contacting the site of the bleeding with said composite dressing.

In some embodiments, the bleeding is caused by surgical or traumatic wounds; and wherein said bleeding is controlled or stopped by application of the dressing to site of bleeding followed by application of pressure.

In other embodiments, the bleeding is controlled or stopped during surgery by:
a) aseptically soaking the composite dressing in a hydration liquid;
b) squeezing the dressing to remove the excess hydration liquid;
c) applying the treated composite dressing at site of the bleeding until hemostasis is achieved.

Furthermore, the present disclosure provides a kit comprising the above described composite dressing and hydration liquid, optionally along with an instruction manual.

### BRIEF DESCRIPTION OF THE ACCOMPANYING FIGURES

In order that the disclosure may be readily understood and put into practical effect, reference will now be made to exemplary embodiments as illustrated with reference to the accompanying figures. The figures together with detailed description below, are incorporated in and form part of the specification, and serve to further illustrate the embodiments and explain various principles and advantages, in accordance with the present disclosure where:
**FIG 1****.** depicts schematic representation of the composite dressing (10) comprising a porous layer (1) and a fabric layer (2). The bonding (3) involves embedding and entanglement of fabric layer (2) into the porous layer (1).
**FIG 2****.** depicts schematic representation of the method of obtaining composite dressing (10). Initially, the biomaterial solution (5) is poured into a hollow mold (4) followed by immersing the fabric support (2) in the biomaterial solution. The biomaterial solution comprising the immersed fabric support is then frozen at a controlled rate to prevent complete dissolution of the fabric support. Thereafter, the complete assembly is dehydrated to obtain the porous matrix (1) bonded onto the fabric support (2).
**FIG 3**. depicts schematic view and representative photograph of a composite dressing (20) comprising a porous matrix head (1) and fabric support as tail (2). The fabric support in the form of a ribbon is provided as Z- fold that can be used as a tether or secondary bandage for porous matrix.
**FIG 4****.** depicts schematic views of a composite dressing (30) comprising a fabric layer (2) sandwiched between two layers of porous matrix (1).
**FIG 5****.** depicts schematic views of a composite dressing (40) comprising a porous matrix (1) sandwiched between two layers of fabric support (2).
**FIG 6****.** depicts schematic views of a tubular composite dressing (50). It comprises a cylindrical porous matrix (1) held in a sleeve of fabric support (2).
**FIG 7****.** (a) depicts scanning electron microscopic (SEM) images of the composite dressing showing the distinct porous layer (1) and the fabric support (2). The fibers of fabric layer can be seen embedded in the porous matrix, (b) depicts that the fabric support of the composite dressing appeared intact, with its fibers embedded within the porous layer. The thickness of the bonding was observed to be about 0.2 mm.
**FIG 8****.** depicts the swelling behavior of the composite dressing after absorbing water. The dressing retains its structure and integrity after saturation with water.
**FIG 9****.** depicts schematic representation of the sample and method used for estimation of bonding strength of fabric support (2) on the porous matrix (1).
**FIG 10****.** depicts hemostatic efficacy of the chitosan-based composite dressing.
**FIG 11****.** depicts structure of chitosan sponges without fabric support (A1 & A2) and chitosan composite dressings (B1 & B2). Top panel shows imaged of dry dressings, while the bottom panel shows dressings being wetted in saline and squeezed to remove excess fluid. The composite dressings retain their smooth texture and integrity (B2), while the standalone chitosan sponge breaks into smaller pieces (A2).
**FIG 12****.** depicts composite dressing prepared using stitching (C1&C2) and by using an adhesive layer between the chitosan sponge and fabric layer (D1 and D2). While the stitching results in breaking of chitosan sponge on wetting (C2), the adhesive layer separates on wetting due change in size of sponge on swelling.
**FIG 13****.** depicts a scanning electron microscopic (SEM) image of the composite dressing of starch and chitosan showing distinct porous matrices of starch attached to the fabric layer of chitosan.
**FIG 14****.** depicts the SEM image of composite hemostatic dressing with an X-ray detectable element sandwiched between the fabric support and chitosan sponge.
**FIG 15****.** depicts photographs and X-ray images of different designs composited dressings containing X-ray detectable element.
**FIG 16****.** depicts photographs and X-ray images of composite dressings containing X-ray detectable element when placed under a piece of meat, wherein the dressings provide sufficient radiopacity to be detected under a piece of meat.
**FIG 17****.** depicts the SEM image of composite hemostatic dressing showing the interaction between fabric support and chitosan sponge in a compressed composite dressing.
**FIG 18****.** depicts the integrity of composite dressing after soaked in saline and squeezed to remove excess saline.
**FIG 19****.** depicts the *in vitro* hemostatic efficacy of composite dressings in both dry and wet form.
**FIG. 20** depicts the reversible bioadhesion between a model of open wound surface and the composite dressing, (a) The composite dressing shows strong bioadhesion when the dressing is applied in the dry form. (b) For removal of the dressing, it is saturated with saline, post-which it can be easily removed.

### DETAILED DESCRIPTION OF THE INVENTION

As used herein, the phrase 'composite dressing' or 'dressing', used interchangeably, refers to wound dressings that combine two or more physically distinct components, such as including but not limiting to layers of porous matrix and fabric support, into a single product, wherein the said components are not combined by use of any adhesive or stitching. Said dressings comprising two or more physically distinct components, are designed to be in direct contact with a wound, which when applied to the wound, promotes healing and/or protects the wound from further harm.

As used herein, the terms 'porous matrix' or 'porous layer' or 'sponge', used interchangeably, refer to the active part of the dressing, formed from controlled freeze-drying or lyophilization of a biomaterial. The controlled freeze-drying herein refers to cooling of biomaterial solution at a constant rate till it freezes and forms uniform ice crystals which leads to formation of uniformly porous and cohesive matrix of the biomaterial.

As used herein, the term 'biomaterial' refers to a natural or synthetic material that is suitable for introduction into or contacting with the living tissue.

As used herein, the phrase 'frozen mass' refers to the frozen biomaterial solution comprising the partially immersed/dissolved fabric substrate.

As used herein, the term/phrase 'fabric', 'fabric support', 'fabric substrate' or 'fabric layer', used interchangeably, refers to the solid support at least partially embedded in the porous matrix of the composite dressing.

Further, usage of the term 'layer' is in congruence with the general meaning of the term, while referring to the porous matrix and/or fabric support of the composite dressing, as appropriate. The term 'interface' and 'bonding layer' used interchangeably refer to the layer wherein the porous matrix and fabric support interact and wherein the fabric support is at least partially embedded in the porous matrix.

Furthermore, reference to the porous matrix 'at least partially embedding' the fabric substrate or fabric support 'at least partially embedded' in the porous matrix, provides for a dressing comprising 1%-100% of the fabric substrate embedded in the porous matrix. However, in the context of the present disclosure, said embedding is such that at least 50% of fibers from the fabric support remain undissolved within or outside the porous matrix. Said embedding of the fabric substrate in the porous matrix, at the interface of the layer of porous matrix and the layer of fabric support is achieved by contacting the fabric substrate with biomaterial solution forming the porous matrix such that the fabric substrate is at least partially immersed in the biomaterial solution such that the fabric substrate at least partially solubilizes and swells in the biomaterial solution. 'Swelling' of the fabric substrate upon contacting with the biomaterial solution forming the porous matrix, refers to partial dissolution and swelling of fibers in the fabric substrate due to the presence of solvent in the biomaterial solution. 'Dry form" of the dressing refers to the final form of the dressing obtained after the step of removal of water from the biomaterial solution to obtain the composite dressing comprising fabric support at least partially embedded in the porous matrix. 'Wet form' of the dressing refers to the final composite dressing saturated with a hydration liquid followed by squeezing out of the excess of hydration liquid.

Accordingly, at least 50% of the fibers from the fabric support/substrate remain undissolved within or outside the porous matrix. The partial dissolution of the fabric substrate in the porous matrix ensures strong bonding between the fabric substrate and the porous matrix at the interface of the two layers wherein said bonding/interaction is not reversible. The resultant bonding or adhesion between the fabric substrate and the porous matrix thus is resistant to separation by mechanical or chemical force.

As used herein, the term 'activated fiber' refers to fibers of the fabric substrate that swell easily in aqueous media as compared to cross-linked fibers. Said 'activated fibers' are obtained by subjecting the fibers to treatment by suitable chemical or physical methods such as but not limiting to acid treatment, crosslinking, exposure to water vapor, heat treatment and gamma irradiation.

As used herein, the term 'absorbance capacity' refers to the capacity of the composite dressing to absorb the fluids such as saline and blood, in terms of the weight of the composite dressing.

As used herein, the phrases 'X-ray detectable element' and 'radio-opaque element' refer to elements incorporated into the dressing, that allow detection by X-ray.

With respect to the use of various well known components in the present disclosure, whose concentrations are not particularly defined herein, said components are deemed to be used in concentrations that are well known to a person skilled in the art in the context of medical/pharmaceutical applications such as those described in the present disclosure.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. The use of the expression 'at least' or 'at least one' suggests the use of one or more elements or ingredients or quantities, as the use may be in the embodiment of the disclosure to achieve one or more of the desired objects or results. Throughout this specification, the word 'comprise', or variations such as 'comprises' or 'comprising' wherever used, will be understood to imply the inclusion of a stated element, integer or step, or group of elements, integers or steps, but not the exclusion of any other element, integer or step, or group of elements, integers or steps.

The present disclosure relates to a composite dressing constituted of one or more biomaterials, wherein the composite dressing comprises at least one layer of porous matrix of the biomaterial and at least another layer of fabric support, wherein the fabric support is at least partially embedded in the porous matrix, as defined in claim 1.

In an embodiment of the present disclosure, the composite dressing is a biomaterial based composite dressing, wherein both the porous matrix and the fabric substrate are composed of biomaterial. In an embodiment, said biomaterial based composite dressing comprises a combination of porous matrix and fabric substrate composed of the same or different biomaterial(s) and wherein the fabric substrate is at least partially embedded in the porous matrix.

In some embodiments of the present disclosure, the composite dressing comprises at least one layer of porous matrix at least partially embedding at least another layer which is a fabric support, wherein both the porous matrix and the fabric support are composed of the same biomaterial. In another embodiment, the composite dressing comprises at least one layer of porous matrix at least partially embedding at least another layer which is a fabric support, wherein both the porous matrix and the fabric support are composed of different biomaterials. In another embodiment, the composite dressing of the present disclosure comprises a combination of porous matrix of a biomaterial and the fabric substrate composed of natural or synthetic fabric materials; wherein the fabric substrate is at least partially embedded in the porous matrix. Accordingly, in an embodiment, only one of the porous matrix and the fabric support are composed of biomaterial. In another embodiment of the present disclosure, the composite dressing comprises at least one layer of porous matrix composed of a biomaterial and at least one layer of fabric substrate composed of natural or synthetic fabric materials. Said natural or synthetic fabric materials may be procured commercially or prepared in the laboratory.

Importantly, the composite dressing of the present disclosure is characterized by cohesive bonding between the porous matrix and the fabric substrate, without use of any adhesive, stitching or chemical crosslinking agent.

The biomaterial(s) forming at least one layer of the composite dressing of the present disclosure is selected from a group comprising chitosan, alginates, starch, cellulose, gelatin, collagen, Polyethylene Glycol (PEG), Polyvinyl Alcohol (PVA), polyurethanes, hyaluronic acid, poly-lysine, poly-glutamic acid, poly(ethylene oxide) (PEO), poly(propylene oxide) (PPO), poly(acrylic acid), poly(methacrylic acid) and their derivatives or any combination thereof. While a person skilled in the art is aware of the derivatives of the said biomaterial(s) that can be employed in the present disclosure, for purposes of exemplification, the derivatives of chitosan include but are not limited to chitosan-PEG, hydrophobically modified chitosan etc., the derivatives of starch include but are not limited to pregelatinized starch and carboxymethyl starch, sodium starch glycolate and the derivatives of cellulose include but are not limited to oxidized cellulose, methyl cellulose and carboxymethyl cellulose.

Accordingly, the present disclosure relates to a composite dressing comprising at least one layer of porous matrix and at least another layer of fabric support, wherein at least one of or both, the porous matrix and the fabric support, are made up of biomaterial(s) selected from a group comprising chitosan, alginates, starch, cellulose, gelatin, collagen, Polyethylene Glycol (PEG), Polyvinyl Alcohol (PVA), polyurethanes, hyaluronic acid, poly-lysine, poly-glutamic acid, poly(ethylene oxide) (PEO), poly(propylene oxide) (PPO), poly(acrylic acid), poly(methacrylic acid) and their derivatives or any combination thereof; and wherein the fabric support is at least partially embedded in the porous matrix.

In a non-limiting embodiment, the composite dressings of the disclosure are composed of a combination of 2-10 biomaterials. For example, the porous matrix may be composed of chitosan in combination with a fabric substrate of alginates, starch, cellulose, gelatin, collagen, Polyethylene Glycol (PEG), Polyvinyl Alcohol (PVA), polyurethanes, hyaluronic acid, poly-lysine, poly-glutamic acid, poly(ethylene oxide) (PEO), poly(propylene oxide) (PPO), poly(acrylic acid), poly(methacrylic acid) and their derivatives or any combination thereof. In another example, the fabric substrate may be composed of chitosan in combination with a porous matrix of alginates, starch, cellulose, gelatin, collagen, Polyethylene Glycol (PEG), Polyvinyl Alcohol (PVA), polyurethanes, hyaluronic acid, poly-lysine, poly-glutamic acid, poly(ethylene oxide) (PEO), poly(propylene oxide) (PPO), poly(acrylic acid), poly(methacrylic acid) and their derivatives or any combination thereof. In an embodiment, the porous matrix and/or the fabric substrate, each, may be composed of a combination of one or more biomaterials selected from a group comprising chitosan, alginates, starch, cellulose, gelatin, collagen, Polyethylene Glycol (PEG), Polyvinyl Alcohol (PVA), polyurethanes, hyaluronic acid, poly-lysine, poly-glutamic acid, poly(ethylene oxide) (PEO), poly(propylene oxide) (PPO), poly(acrylic acid), poly(methacrylic acid) and their derivatives. In a preferred embodiment, the composite dressing of the present disclosure comprises a fabric substrate composed of chitosan, in combination with a porous matrix of starch.

In another embodiment of the present disclosure, while the porous matrix is made up of biomaterial(s) selected from a group comprising chitosan, alginates, starch, cellulose, gelatin, collagen, Polyethylene Glycol (PEG), Polyvinyl Alcohol (PVA), polyurethanes, hyaluronic acid, poly-lysine, poly-glutamic acid, poly(ethylene oxide) (PEO), poly(propylene oxide) (PPO), poly(acrylic acid), poly(methacrylic acid) and their derivatives or any combination thereof; the fabric support at least partially embedded in the porous matrix is prepared from natural material selected from a group comprising cotton, cellulose and/or its derivatives, wool, silk and blends thereof, or synthetic material selected from a group comprising polyamide, polyester, rayon and blends thereof.

In an embodiment, the fabric substrate is a rayon-polyester blended gauze wherein the ratio of rayon to polyester ranges from about 0:100 to about 100:0, preferably about 25:75 to about 75:25.

Further, without wishing to be bound by any particular theory, the composite dressing of the present disclosure may employ as biomaterial, any polymer, any synthetic material or any natural material in addition to those exemplified above, which dissolves in a solvent employed in the present disclosure and forms a porous matrix when the resultant solution is subjected to lyophilization.

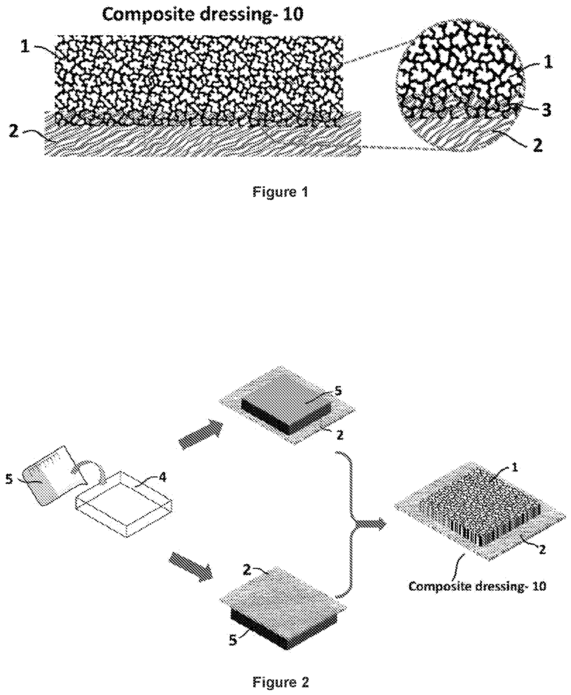

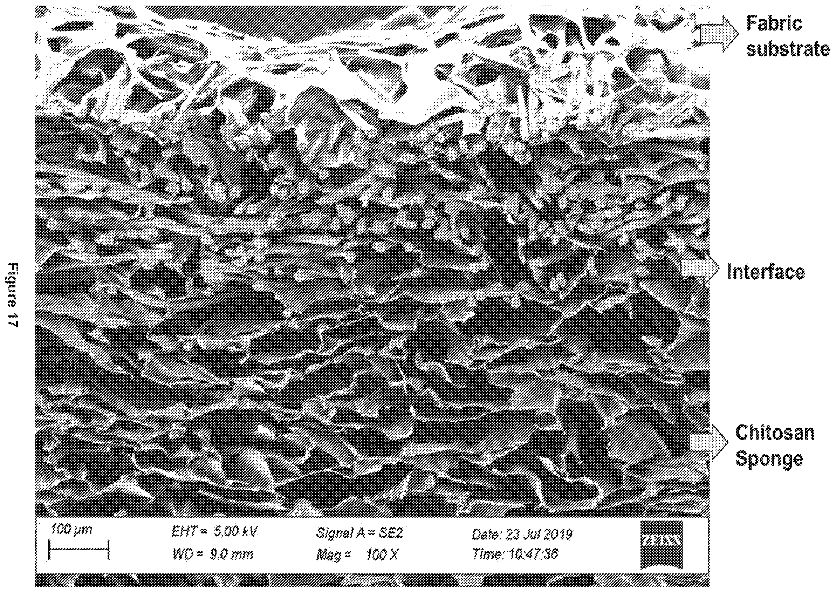

Figure 1 depicts schematic representation of the composite dressing (10) comprising a porous layer (1) and a fabric layer (2). The bonding (3) involves embedding and entanglement of fabric layer (2) into the porous layer (1), The porous matrix is the active part of the dressing, while the fabric substrate serves one or more of the following functions - a non-adherent backing layer, a reinforcing member for the porous matrix, a tether for easy placement and removal of porous matrix in deep wounds, or a secondary bandage to hold porous matrix in place. Further, Figure 17 depicts the SEM image of composite hemostatic dressing showing the interaction between fabric support and chitosan sponge in a compressed composite dressing.

In some embodiments of the present disclosure, the porous matrix of the composite dressing has high porosity, wherein pore size ranges from about 10 µm to about 300 µm and the average pore size is about 100 µm. Said high porosity of the porous matrix allows efficient fluid absorption at the wound site, on application of the dressing.

In an embodiment, thickness of the porous matrix layer in the composite dressing is at least 1 mm. In another embodiment, the thickness of the porous matrix layer in the composite dressing ranges from about 1 mm to about 100 mm, preferably from about 5 mm to 10 mm.

Further, wall thickness of the porous matrix of the composite dressing is at least 0.1 µm. In a non-limiting embodiment, the wall thickness of the porous matrix of the composite dressing ranges from about 0.05 µm to about 5 µm, preferably from about 0.5 µm to about 1µm.

In another non-limiting embodiment, the fabric substrate of the composite dressing is a fibrous material, wherein said fabric substrate is in the form of support material selected from a group comprising gauze, bandage and a patch or ribbon of fabric composed of the aforementioned biomaterials. The fabric substrate gives the mechanical strength for handling the dressing. The length of fibers in the fabric substrate is at least 1 mm, more preferably ranging from about 10 mm to about 100 mm.

The fabric substrate is selected such that complete dissolution of the fabric substrate is prevented during preparation of the composite dressing. In a non-limiting embodiment of the disclosure, thickness of the fabric substrate ranges from about 0.05 mm to about 5 mm, preferably from about 1 mm to about 2 mm. The weight of the fabric substrate is at least about 1 gram per square meter (gsm), preferably ranging from about 50 gsm to about 500 gsm, more preferably ranging from about 90 gsm to about 150 gsm. Fiber density of the fabric substrate is at least about 10%, preferably ranging from about 25% to about 50%. Linear density of the fabric substrate is at least about 0.1 dtex (decitex), preferably ranging from about 1.6 dtex to about 2 dtex. Said fabric substrate is selected such that it has fluid absorption ability of at least 5 times, preferably about 15 times to about 40 times its dry weight. Further, the fabric substrate has dry breaking strength of at least about 0.1 MPa (megapascal), preferably ranging from about 1.4 MPa to about 3.5 MPa; and wet breaking strength of at least about 0.01 MPa, preferably ranging from about 0.5 MPa to about 2 MPa. Furthermore, for both dry and wet fabric substrates, minimum elongation at break is at least about 5%, preferably ranging from about 50% to about 200% of the original length. Said elongation as break is depictive of the elastic properties allow the fabric support to stretch/ shrink with changes in size of attached sponge during application.

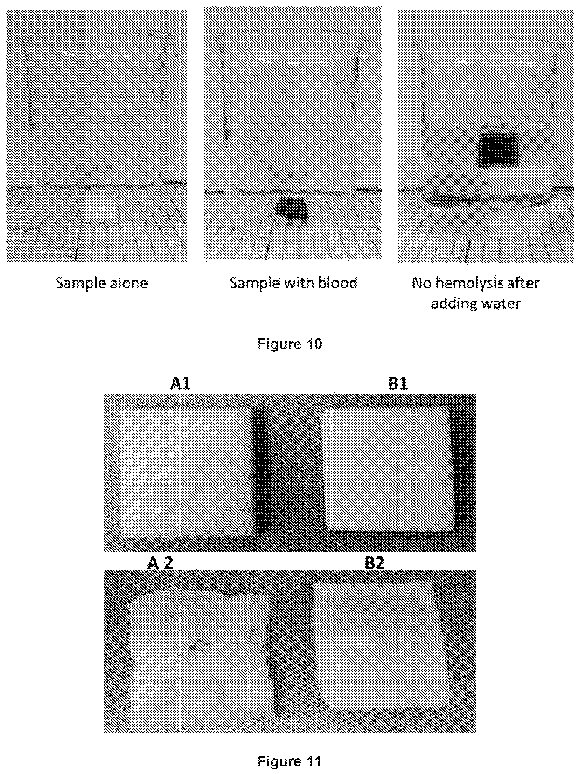

The fabric substrate confers high tensile strength to the composite dressing of the present under both dry and wet conditions. Under wet conditions, the composite dressings retain their smooth texture and integrity as compared to standalone adsorbent porous matrices which break into smaller pieces as shown in Figure 11.

The bonding between the porous layer and the fabric substrate in the composite dressing of the disclosure is cohesive, wherein thickness of the bonding between the layer of porous matrix and the layer of fabric support, at their interface, is at least about 1% of the thickness of the fabric substrate, preferably ranging from about 10% to about 1 00% of the thickness of the fabric substrate. Said thickness is indicative of the depth to which the biomaterial solution infiltrates the surface of the fabric substrate.

Further, the composite dressing of the present disclosure may have different design variants.

In some embodiments of the present disclosure, the composite dressing is designed with the fabric substrate at the bottom of the porous matrix.

In an embodiment, the composite dressing comprises alternating layers of the fabric substrate and the porous matrix.

In a further embodiment, the composite dressing comprises 3 layers; wherein the fabric substrate is sandwiched between two layers of the porous matrix or the porous matrix is sandwiched between two layers of the fabric substrate.

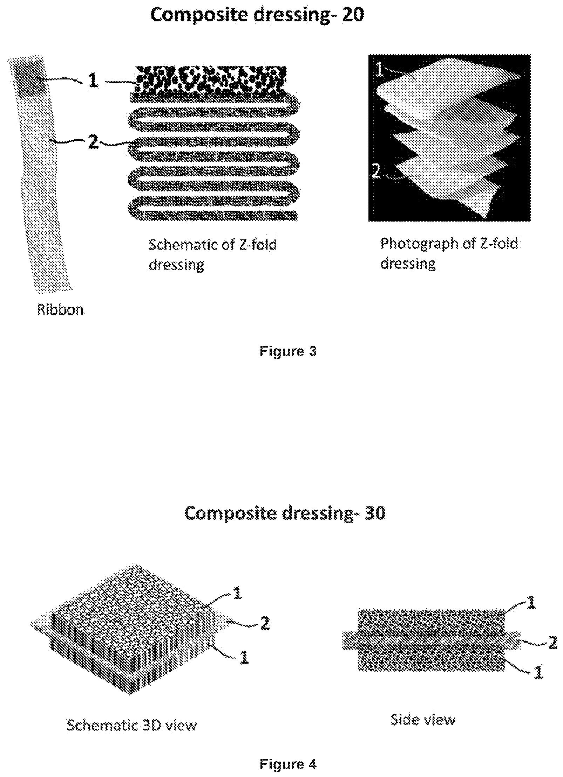

In an embodiment, the composite dressing is designed with the fabric substrate in the center of two layers of the porous matrix, as exemplified in Figure 4.

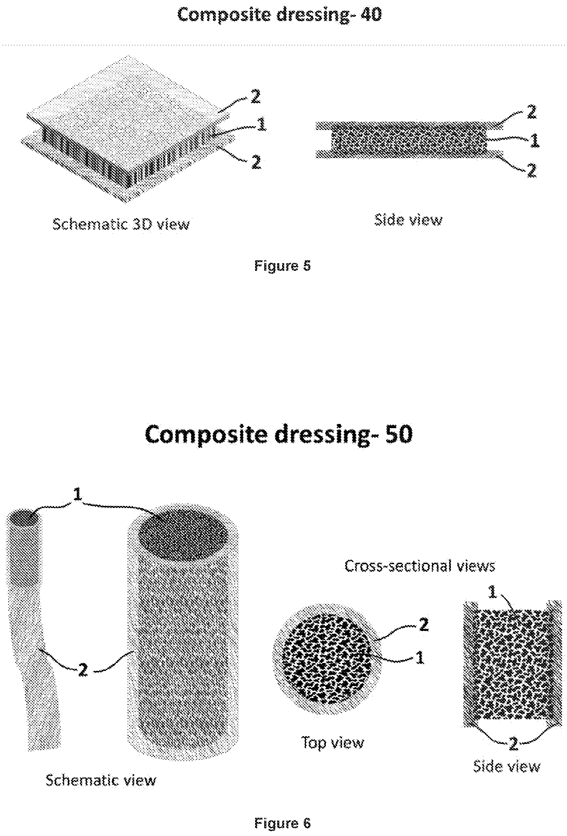

In yet another embodiment, the composite dressing is designed with the porous matrix sandwiched between two layers of the fabric substrate, as exemplified in Figure 5.

In a further embodiment, the composite dressing is designed with the porous matrix attached to a long string of fabric substrate, as exemplified in Figure 3. Said string of fabric is longer than the porous matrix. The longer string of the fabric support acts as a tether for the porous matrix to be pulled out from application site such as uterine cavity (PPH) or deep cavity wounds.

In an embodiment, said long string of fabric substrate is folded into a zig-zag fold and one end of the string of fabric substrate is attached to the porous matrix.

In another embodiment, the composite dressing is designed as a tubular composite dressing with cylindrical porous matrix wrapped in tubular fabric substrate, as exemplified in Figure 6. Said tubular composite has diameter ranging from about 0.1 cm to about 10 cm.

In a still further embodiment, the composite dressing, in any of the configurations as described above, is designed with the fabric substrate impregnated with a radio-opaque filament for easier tracking using X-ray after implantation within the body. Said dressing provides sufficient radiopacity, allowing easy detection post implantation.

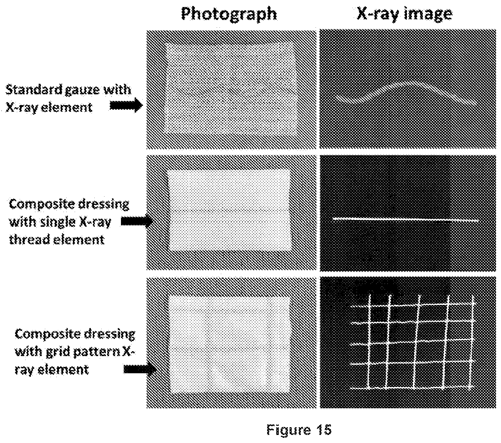

In an embodiment, fabric substrate contains an X-ray detectable thread. Said thread is configured into the fabric substrate in a form such as but not limiting to a single line or multiple thread in random or ordered orientation. In an embodiment, the multiple threads are configured in a parallel or in a grid arrangement. Placing the X-ray detectable thread in a grid pattern helps in reinforcing the porous matrix to improve its strength. Figure 15 shows the different arrangements of X-ray detectable thread in the composite dressing.

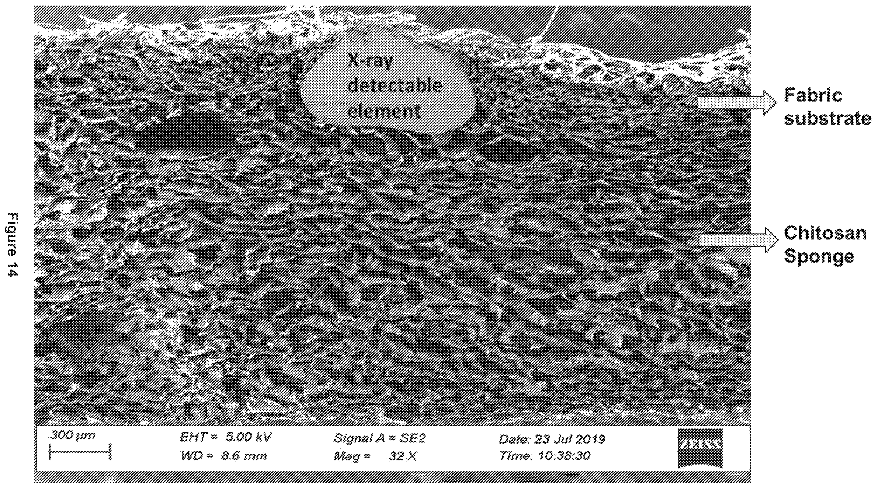

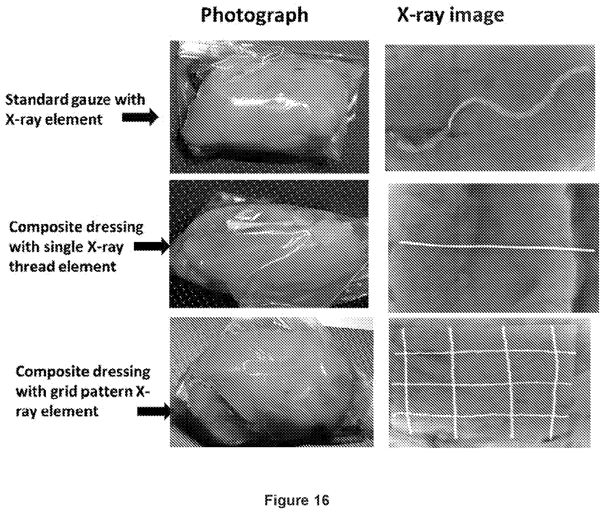

In a further embodiment, said x-ray detectable thread is pre-attached to the fabric substrate. Said pre-attachment if performed by methods such as but limiting to heat bonding or by sewing. In another embodiment, an external x-ray detectable element such as thread is sandwiched between the fabric substrate and the chitosan solution. After lyophilization, the thread is firmly held between the fabric substrate and the lyophilized sponge as depicted in Figure 14. Figure 16 shows the radiopacity provided by the different exemplary configurations of composite dressings comprising X-ray detectable elements.

With regard to stability and strength of the composite dressing of the present disclosure, the dressing shows good stability in presence of aqueous solutions or biological fluids. The strong bonding between the porous layer and the fabric support provides excellent peel strength in both dry and wet conditions. Importantly, the composite dressing has a significantly high peel strength, higher than the bioadhesion force between the porous layer and tissues, which provides for easier removal of the composite dressings from wounds, body cavities without leaving any residue. In an embodiment, the bonding/peel strength between the porous matrix and the fabric support is higher than the individual tensile strengths of fabric substrate or the porous matrix.

In a non-limiting embodiment, the bonding strength between the porous matrix and the fabric support is considerably higher than the individual tensile strengths of fabric support or porous matrix. This is achieved by the at least partial embedding and partial dissolution of fabric support in the porous matrix, leading to a very strong bonding between the two layers- i.e. the layer of porous matrix and the layer of fabric support. The resultant bonding or adhesion between the fabric substrate and the porous matrix thus is resistant to separation by mechanical or chemical force.

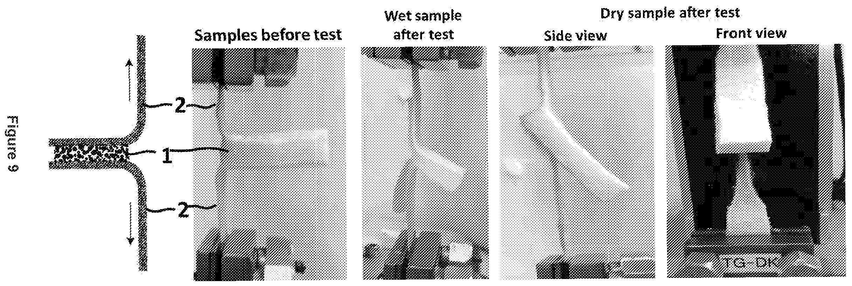

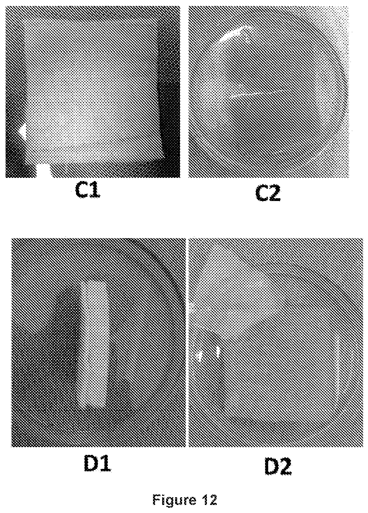

Figure 9 depicts schematic representation of the sample and images of samples used for estimation of bonding strength between fabric support (2) and the porous matrix (1). As shown, during the bonding strength measurement experiment, the bonding between the layers remained intact in both dry and wet conditions. When a composite dressing is prepared by adhesive or stitching, the fabric support restricts this swelling which results in either separation of layers of breaking of sponge, as shown in Figure 12. On the other hand, the composite dressings of the present disclosure allow the porous matrix (such as chitosan sponge) to swell without separation as the fibers of fabric support are deeply embedded in the matrix.

The bonding strength or peel strength between fabric support and porous layer is controlled by the composition of fabric support, extent of interaction between the two layers, wettability of fabric support and the tensile strength of the fabric support.

The bonding thickness at the interface of the fabric substrate and porous matrix is also dependent on the biomaterial solution viscosity and freezing time. In a non-limiting embodiment, the bonding thickness can be increased by reducing the viscosity of the biomaterial solution as well as by increasing the contact duration between the fabric and solution before freezing or using a slow freezing cycle. In an embodiment, viscosity of the biomaterial is adjusted by controlling/modulating temperature of the biomaterial solution. In an embodiment, the viscosity of the biomaterial solution is maintained at >1000 cP.

In an embodiment of the present disclosure, moisture content of the composite dressing ranges from about 5% to about 25%.

In an embodiment, pH of the composite dressing composed of 100% chitosan ranges from about 4 to about 7.

In another non-limiting embodiment, tensile strength of the composite dressing ranges from about 0.5 MPa to about 10 MPa in dry conditions and about 0.1 MPa to about 5 MPa in wet conditions.

In a further non-limiting embodiment, elongation at break of the composite dressing ranges from about 5% to about 20% in dry conditions and about 10% to about 50% in wet conditions. Further, the bend angle of the composite dressing ranges from about 90 degrees to about 180 degrees. Preferably, bend angle of the composite dressing of the present disclosure is about 180 degrees.

Said bend angle depicts the flexibility of the dressing allowing it to be folded or rolled as per requirement.

In another non-limiting embodiment, the composite dressing has absorbance capacity ranging from about 10 times to about 200 times its original weight.

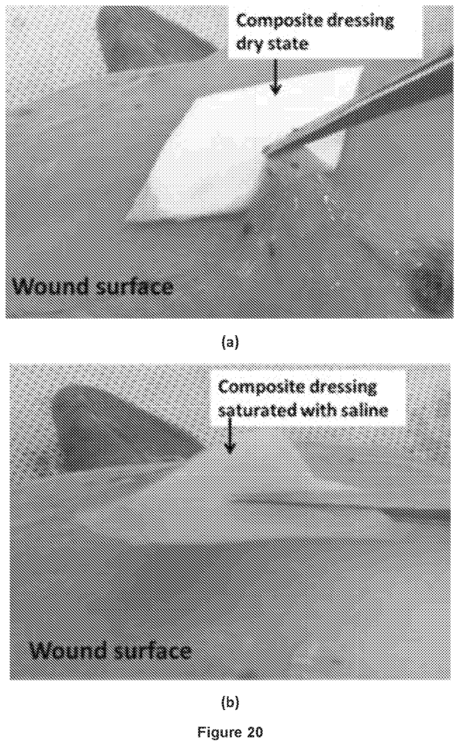

In an exemplary embodiment of the present disclosure, the adhesion force of the dressings to the wound tissue ranges from about 0.1 MPa to 1 MPa Said adhesion occurs between the wound or site of bleeding and the porous matrix side of the composite dressing, as shown in Figure 20.

As mentioned previously, one of the crucial aspects of the present disclosure lies in the manner in which the porous matrix and the fabric substrate of the composite dressing of the present disclosure interact with each other. This interaction by way of cohesive bonding and without use of any adhesive, stitching or chemical crosslinking agent is achieved by the way the two components come together. In other words, the composite dressing of the present disclosure is prepared by a unique method that allows the optimum interaction by way of partial embedding of the fabric support by the porous matrix. This method is therefore also important for achieving the beneficial properties described above.

Thus, the present disclosure also provides a method for preparing the composite dressing described above, wherein said method comprises:
a) contacting the fabric substrate with a solution of the biomaterial;
b) at least partially immersing the fabric substrate in the solution of biomaterial;
c) freezing the solution of biomaterial comprising the immersed fabric substrate at a controlled rate; and
d) removing solvent from the frozen biomaterial solution of (c),
   to obtain the composite dressing comprising the porous matrix at least partially embedded with the fabric substrate.

The method described in the present disclosure combines formation of the porous matrix and bonding of said porous matrix with the fabric substrate into a single step. Figure 2 depicts schematic representation of the method of obtaining composite dressing-10. Initially, the biomaterial solution (5) is poured into a hollow mold (4) followed by immersing the fabric support (2) in the biomaterial solution. The biomaterial solution comprising the immersed fabric support is then frozen at a controlled rate to prevent complete dissolution of the fabric support. Thereafter, the complete assembly is dehydrated to obtain the porous matrix (1) bonded onto the fabric support (2).

In an embodiment, the porous matrix and the fabric substrate are composed of same or different material.

In another embodiment, one or both of the porous matrix and the fabric substrate are composed of biomaterial(s).

In some embodiments of the present disclosure, the porous matrix and the fabric substrate are composed of same or different biomaterial(s), wherein said biomaterial(s) is selected from a group comprising chitosan, alginates, starch, cellulose, gelatin, collagen, Polyethylene Glycol (PEG), Polyvinyl Alcohol (PVA), polyurethanes, hyaluronic acid, poly-lysine, poly-glutamic acid, poly(ethylene oxide) (PEO), poly(propylene oxide) (PPO), poly(acrylic acid), poly(methacrylic acid) and their derivatives or any combination thereof. While a person skilled in the art is aware of the derivatives of the said biomaterial(s) that can be employed in the present disclosure, for purposes of exemplification, the derivatives of chitosan include but are not limited to chitosan-PEG, hydrophobically modified chitosan etc., the derivatives of starch include but are not limited to pregelatinized starch, carboxymethyl starch, sodium starch glycolate etc. and derivatives of cellulose include but are not limited to oxidized cellulose, methyl cellulose, carboxymethyl cellulose etc. Without wishing to be bound by any particular theory, the composite dressing of the present disclosure may employ as biomaterial, any polymer, in addition to those exemplified above, which dissolves in a solvent employed in the present disclosure and forms a porous matrix when the resultant solution is subjected to lyophilization.

In a preferred embodiment, the biomaterial is chitosan of molecular weight of at least 1 kDa, more preferably chitosan of molecular weight ranging from about 200 kDa to about 400 kDa.

In another embodiment of the present disclosure, while the porous matrix is made up of biomaterial(s) selected from a group comprising chitosan, alginates, starch, cellulose, gelatin, collagen, Polyethylene Glycol (PEG), Polyvinyl Alcohol (PVA), polyurethanes, hyaluronic acid, poly-lysine, poly-glutamic acid, poly(ethylene oxide) (PEO), poly(propylene oxide) (PPO), poly(acrylic acid), poly(methacrylic acid) and their derivatives or any combination thereof; the fabric support, at least partially embedded in the porous matrix, is prepared from natural material selected from a group comprising cotton, cellulose and/or its derivatives, wool, silk and blends thereof, or synthetic material selected from a group comprising polyamide, polyester, rayon and blends thereof, before contacting with the biomaterial solution. Said pre-treatment is separate from the preparation of special fabric. In an embodiment, the special fabric obtained by mixing fibers having wetting and swelling properties different from each other is subjected to pre-treatment prior to preparing the composite dressing.

In some embodiments of the present disclosure, prior to contacting with the biomaterial solution, the fabric substrate is pre-treated by exposure to elevated temperatures ranging from about 40°C to about 90°C for time period ranging from about 1 hour to about 6 hours, to reduce dissolution in the biomaterial solution.

In another embodiment of the present disclosure, the fabric substrate is pre-treated by contacting with surfactant or plasticizer. One or more surfaces of the fabric substrate may be treated with a surfactant or plasticizer solution either by dip coating or spray coating to improve its wettability and absorbency characteristics, wherein the surfactant or the plasticizer is selected from a group comprising poloxamer, polysorbates (such as polysorbate 20 and polysorbate 80), betaine, lecithin, lauryl sulphate, Pluronic F-68, Sorbitantrioleate, d-α-tocopherol polyethylene glycol 1000 succinate, Polyethoxylated castor oil, polyethoxylated 12-hydroxystearic acid, glycerol, polyethylene-glycol (PEG), ethylene glycol, sorbitol, polyvinyl pyrrolidone (PVP), polyvinyl alcohol (PVA), ethylene glycol and propylene glycol or any combination thereof.

In another embodiment, the active agent is selected from a group comprising pro-coagulant and natural, semisynthetic or synthetic sealant or any combination thereof. The pro-coagulant is further selected from a group comprising calcium chloride, calcium silicate nanoparticles, Factor X activators, prothrombin activators, fibrinogen, vitamin K, thrombin, platelet rich plasma, fibrin, tranexamic acid and aminocaproic acid or any combination thereof, and the sealant is surgical sealant selected from a group comprising thrombin-fibrinogen based sealant, gelatin-thrombin, albumin and thrombin solution or any combination thereof. Said sealant is in liquid or semisolid form.

Pre-treatment of the fabric substrate leads to improved surface properties of the fabric substrate. The composite dressings made using said pre-treated substrates have a smoother surface, uniform pore size, better flexibility and better wet strength as compared to dressings made from porous matrices alone, without the fabric substrate.

In some embodiments of the present disclosure, the fabric substrate is composed of a special fabric obtained by mixing fibers having wetting and swelling properties different from each other. To achieve these, raw fibers of biomaterials such as but not limited to chitosan, alginate collagen, or cellulose are crosslinked by either chemical or physical crosslinking methods to increase their wet strength, wettability and control their swelling properties and their susceptibility to dissolution in biomaterial solution. The chemical crosslinking methods include but are not limited to treatment with crosslinking agents such as but not limited to glutaraldehyde, genipin, calcium chloride, epichlorohydrin, carbodiimide derivatives, N-hydroxysuccinimide esters and combinations thereof. Physical crosslinking methods involve heating the fibers at elevated temperature under controlled humidity. In a second approach, the fibers of the fabric substrate are activated by treatment of the fabric substrate by suitable chemical or physical methods such as but not limiting to acid treatment, exposure to water vapor, heat treatment, gamma irradiation etc. to yield activated or non-crosslinked fibers in order to increase their swelling properties or dissolution in aqueous media. Said cross-linked fibers and activated or non-crosslinked fibers are further combined to yield the mixed fabric substrate. The mixed fabric substrate may be a woven or non-woven fabric. In an embodiment, said cross-linked fibers and non-crosslinked or activated fibers are combined by methods such as but not limited to processing through a needle punching apparatus.

In a non-limiting embodiment, the cross-linked fibers are prepared by contacting about 10% w/v to about 20% w/v of the fibers of fabric substrate with about 0.5 % w/v to about 10% w/v , preferably about 1% w/v of cross-linking agent for about 1 hour to about 48 hours, preferably about 24 hours, followed by washing and drying to obtain the cross-linked fibers. In another non-limiting embodiment, the activated or non-crosslinked fibers are prepared by contacting about 10% w/v to about 20% w/v of the fibers of fabric substrate with about 0.1 % w/v to about 10 % w/v, preferably about 1% v/v of acid selected from a group comprising acetic acid, lactic acid, succinic acid, glycolic acid etc. followed by mixing, recovering the treated fibers from the suspension by methods such as but not limiting to filtration and drying.

Further, in another non-limiting embodiment, about 1 0% w/w to about 50% w/w of the crosslinked fibers are mixed with about 50% w/w to about 90% w/w of non-crosslinked or activated fibers to obtain the fabric of mixed stability and swelling properties. For example, the fabric substrate with mixed fiber may have composition such as but not limiting to 10 % crosslinked fiber + 90% non-crosslinked fiber, 30 % crosslinked + 70 % non-crosslinked fiber and 50% crosslinked + 50% non-crosslinked fiber.

To provide further exemplification of the preparation of the mixed fiber fabric substrate described above, for the preparation of a chitosan based fabric substrate with mixed fibers, about 10 % w/v to about 20% w/v chitosan fibers are suspended in water containing about 1% w/v glutaraldehyde for about 24 hours. After the glutaraldehyde treatment, the fibers are washed with water and dried at a temperature of about 60 °C. Further, non-crosslinked or acid-treated chitosan fibers are prepared by suspending about 10% w/v to about 20% w/v chitosan fibers in ethanol and adding about 1% v/v acetic acid to said suspension followed by mixing for about 0.5 hours to 3hours, preferably for 1 hours at room temperature and recovery of the acid treated or non-crosslinked chitosan fibers from the suspension by filtration and drying at about 60°C for about 4 hours. The mixed fiber fabric substrate is then prepared by mixing or combining about 10% w/w to about 50% w/w of the crosslinked fibers with about 50% w/w to about 90% w/w acid-treated chitosan fibers and processing through a needle punching apparatus to obtain a non-woven fabric.

When such a mixed fiber fabric substrate is used in preparation of composite dressings of the present disclosure, the activated or non-crosslinked fibers partially dissolve and swell in the biomaterial solution, while the cross-linked fibers remain stable resulting in better bonding strength between the layers.

In a non-limiting embodiment, the pre-treated fabric substrate and the fabric substrate with mixed fiber have improved tensile strength, wettability and solubility in biomaterial solution as compared to untreated or conventional fabric substrate.

As mentioned previously, the method of manufacturing the composite dressings of the present disclosure involves contacting the fabric substrate with the solution of the biomaterial. This is achieved by carefully placing the fabric substrate under the solution of the biomaterial or mounting the fabric substrate on top of the solution of the biomaterial. The fabric substrate is contacted with the solution of biomaterial in a mold or tray such that at least about 1% of the thickness of fabric, preferably ranging from about 10% to about 100% of fibers of the fabric substrate are wetted by the solution of the biomaterial.

In some embodiments of the present disclosure, immersing the fabric substrate in the solution of biomaterial partially dissolves the fabric substrate.

After immersing the fabric substrate in the solution of biomaterial, said solution of biomaterial comprising the at least partially immersed/partially dissolved fabric support is subjected to freezing by reducing the temperature of biomaterial solution at controlled rate. Temperature of the biomaterial solution is reduced to about -20°C to about 0°C at a freezing rate of about 0.1°C to about 5°C per minute. The solution was then maintained at said temperature for time duration ranging from about 0.5hours to about 12 hours, wherein the rate of freezing is adjusted to prevent complete dissolution of fabric support in the biomaterial solution.

In some embodiments of the present disclosure, the rate and duration of freezing ensures that minimum 50% of fibers from the fabric support remain undissolved.

After freezing, solvent from the frozen mass of biomaterial solution comprising the at least partially immersed/partially dissolved fabric substrate is removed by a method which ensures sublimation of water molecules directly from the frozen mass. In a non-limiting embodiment, water is removed from the frozen mass by freeze-drying/lyophilization or dipping of the frozen mass in dehydrating solvent, wherein the dehydrating solvent is selected from a group comprising organic solvents such as alcohol, acetone, ethylacetate, or any combination thereof.

Said dehydrating organic solvents are selected such that they are miscible with water, while the biomaterial is insoluble in the organic solvents. Therefore, after repeated washings with acetone or alcohol a dry porous matrix of biomaterial such as a sponge can be obtained. The process can be reversed for a biomaterial which is soluble in the organic solvents. In such cases, the frozen mass of biomaterial can be dipped in an aqueous solution to remove the organic solvent.

In a non-limiting embodiment, the frozen biomaterial solution comprising the at least partially immersed/partially dissolved fabric substrate is subjected to lyophilization at predefined lyophilization cycle, wherein, first the chitosan solution is frozen at a controlled rate as described above, second the frozen solution is exposed to a controlled vacuum of about 50 mTorr to about 250 mTorr at temperatures ranging from about -40°C to about 15°C for about 8 hours to about 36 hours. Third, secondary drying is carried out at temperatures ranging from about 15°C to about 35°C for about 2 to about 10 hours.

Removal of solvent by the above defined method(s) results in formation of a porous matrix of the biomaterial with well-defined pore size and high porosity, wherein pore size ranges from about 10 µm to about 300 µm and the average pore size is about 100 µm. In a non-limiting embodiment, wall thickness of the porous matrix is at least about 1 µm. Said porous matrix is embedded with the fabric support such that minimum of about 50% of fibers from the fabric support remain undissolved within or outside the porous matrix.

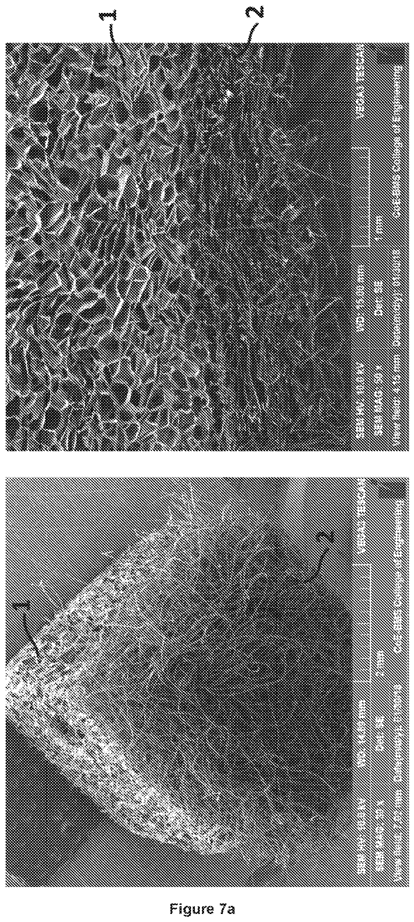

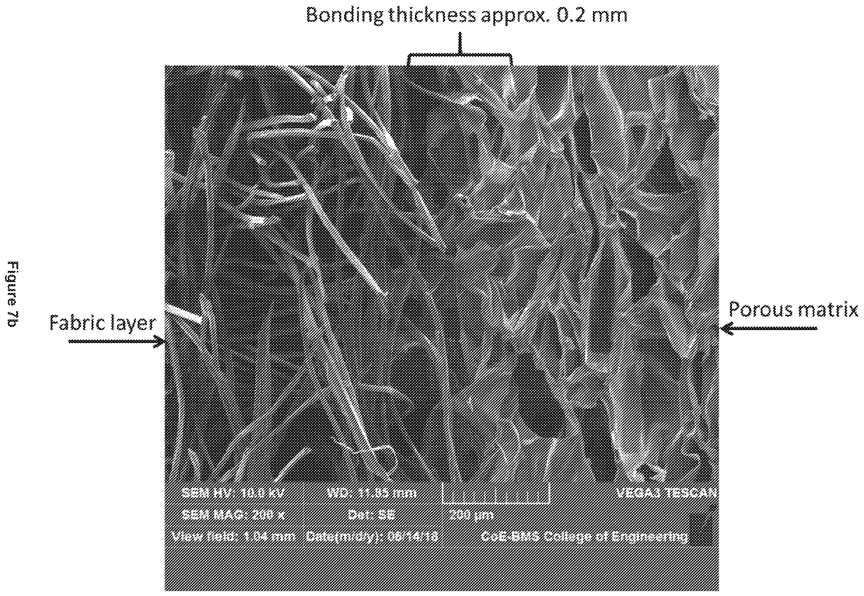

The above described method thus results in formation of a composite dressing comprising distinct porous matrix of the biomaterial embedded with the fabric support. The method provides a multi-layered dressing of same or different biomaterial, such that at least one layer is a porous matrix of the biomaterial embedded with at least another layer which is a fabric support of same or different biomaterial; wherein the porous matrix is the active part of the dressing, while the fabric substrate serves one or more of the following functions- a non-adherent backing layer, a reinforcing member for the porous matrix, a tether for easy placement, removal of porous matrix in deep wounds or a secondary bandage to hold porous matrix in place. The fabric substrate confers high tensile strength to the composite dressing of the present disclosure under both dry and wet conditions. Under wet conditions, the composite dressings retain their smooth texture and integrity as compared to standalone adsorbent porous matrices which break into smaller pieces as shown in Figure 11. Figure 7 depicts scanning electron microscopic (SEM) images of the composite dressing showing the distinct porous layer (1) and the fabric support (2). The fibers of fabric layer can be seen embedded in the porous matrix.

In some embodiments of the present disclosure, different variants of composite dressing can be obtained by altering the placement of gauze dressing in the mold or tray. Such variants include but are not limited to;
i. composite dressing with the fabric substrate at the bottom of the porous matrix,
ii. composite dressing with the fabric substrate sandwiched between two layers of the porous matrix, wherein said dressing has applications such as those in management of bleeding from body cavities such as nasal bleeding or hemorrhoidal bleeding; or
iii. composite dressing with a porous matrix sandwiched between two layers of the fabric substrate, wherein such a dressing could be used for management of bleeding after sinus surgery and preventing the tissue adhesions. The two layers of fabric support ensures complete removal of the porous matrix from the body cavities.

In another embodiment, the composite dressing comprises a porous matrix attached to a long string of fabric substrate (Figure 3). In a non-limiting embodiment, length of the string ranges from about 0.5 meter to 3 meters.

In order to obtain another variant of the composite dressing, the long string fabric substrate is folded into a zig-zag fold. The folded fabric substrate is then placed on top of the biomaterial solution in a way that only the bottom layer of the zig-zag folded fabric substrate interacts with the biomaterial solution. The resulting composite dressing contains a porous matrix head attached to a long tail of fabric substrate i.e. the porous matrix attached to one end of the long tail of the fabric substrate. The benefit of such a dressing is that it can be used for filling a deep cavity wound with the porous matrix, without any concern for leaving residue at the wound site. The extended fabric support acts as a tether for the removal of porous matrix. The ribbon of fabric support can also be used as a secondary bandage to wrap the porous matrix around the wound.

In another embodiment, the fabric substrate used for preparing the composite dressings herein is impregnated with a radio-opaque filament for easier tracking using X-ray after implantation within the body.

In another embodiment, tubular composite dressings are prepared (Figure 6) by using cylindrical molds; wherein a tubular fabric substrate is lined inside a cylindrical mold and biomaterial solution is poured inside the tubular fabric substrate. The resulting composite dressing comprises a cylindrical porous matrix wrapped in a tubular fabric substrate. Such a dressing can be used in management of bleeding from gun-shot wounds. The extended tube of fabric support helps in pulling out the dressing after hemostasis is achieved.

Illustration of examples of variants of the composite dressing of the present disclosure are provided in Figures 3-6.

The composite dressing of the present disclosure having distinct porous matrix and fabric support are prepared such that the porous matrix in the composite dressing is neither compressed nor affected by the preparation method, since the porous matrix is not subjected to any processes tending to deform or shrink it in any direction. Similarly, the fabric layer retains its internal fiber structure and texture after combining with the porous matrix since complete dissolution of the fabric layer in the porous matrix of the composite dressing is prevented.

In a non-limiting embodiment, the method of the present disclosure ensures that minimum 50% of fibers from the fabric substrate remain undissolved and outside the porous matrix.

In an embodiment of the present disclosure, the method of preparing the composite dressing further comprises saturating the composite dressing with hydration liquid selected from a group comprising water, saline, pro-coagulant material and natural, semisynthetic or synthetic sealants or any combinations thereof, followed by squeezing out excess liquid, to obtain wet form of the composite dressing.

In an embodiment, the pro-coagulant is selected from a group comprising calcium chloride, calcium silicate nanoparticles. Factor X activators, prothrombin activators, fibrinogen, vitamin K, thrombin, platelet rich plasma, fibrin, tranexamic acid and aminocaproic acid or any combination thereof; the sealant is surgical sealant selected from a group comprising thrombin-fibrongen based sealants, gelatin-thrombin, albumin and thrombin solution or any combination thereof. Said sealant is in liquid or semisolid form.

The application in wet form allows use of the composite dressing in same manner as the conventional gelatin foams. The process of saturating the composite dressing with hydration liquid followed by squeezing out the excess liquid softens the dressing and allows for faster blood absorption and close contact with the wound site. Said dressing is highly flexible and can be rolled into the form of a tube having diameter ranging from about 1mm to about 10mm. Said wet form of the composite dressing also provides ease of application to irregular surfaces such as orthopedic surgeries, anastomosis sites. The flexibility of the dressing allows it to be wrapped around an anastomosis site.

In a still further embodiment of the present disclosure, the method of preparing the composite dressing further comprises compressing the composite dressing; wherein the compressing is performed by techniques selected from a group comprising but not limited to roller milling and hydraulic press. The final thickness of said compressed composite dressings ranges from about 0.1 mm to about 0.3 mm. Said step of compression of the composite dressings increases the flexibility and ease of application to wound site. The compressed dressings are flexible enough to be rolled into a tube or cylinder shape having diameter ranging from about 1 mm to about 10 mm, preferably about 2 mm to about 3 mm diameter. Said properties allow administration of the composite hemostatic dressings through narrow channels of endoscope or laparoscope for use during laparoscopic surgeries.

The composite dressing of the present disclosure despite comprising no adhesive between the porous matrix and the fabric support shows high bonding strength between the two layers, preventing detachment of said two layers during application.

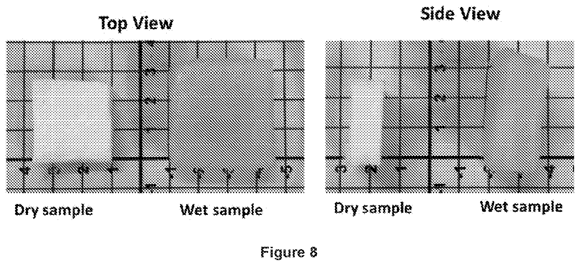

Further, the composite dressing shows high fluid absorption or swelling behaviour. Figure 8 depicts swelling behavior of the composite dressing after absorbing water. The dressing retains its structure and integrity after saturation with water. Figure 10 further depicts the hemostatic efficacy of the composite dressing of the present disclosure.

In a non-limiting embodiment, the composite dressing composed of chitosan shows fluid absorption of at-least about 20 times its weight, more specifically between about 30 to about 60 times its weight, while the porous matrix of the dressing swells to at-least about 1.5 times its original size on fluid absorption. Said composite dressing composed of chitosan achieves complete hemostasis in about 3 minutes to about 7 minutes.

The composite dressing of the present disclosure has multiple applications in the medical field. One such exemplary application is in emergency hemostasis in cases of traumatic injuries. The combination of porous matrix and fabric substrate provides an improved hemostatic dressing suitable for controlling bleeding in deep cavities or body orifices such as seen in traumatic injuries or post-partum hemorrhage. The excellent mechanical properties of the composite dressing make it fit for applications such as hemostatic dressings in surgical bleeding and internal hemostasis. Other applications of such dressings include the use of the dressings as body implants to repair or reinforce soft tissue and organs.

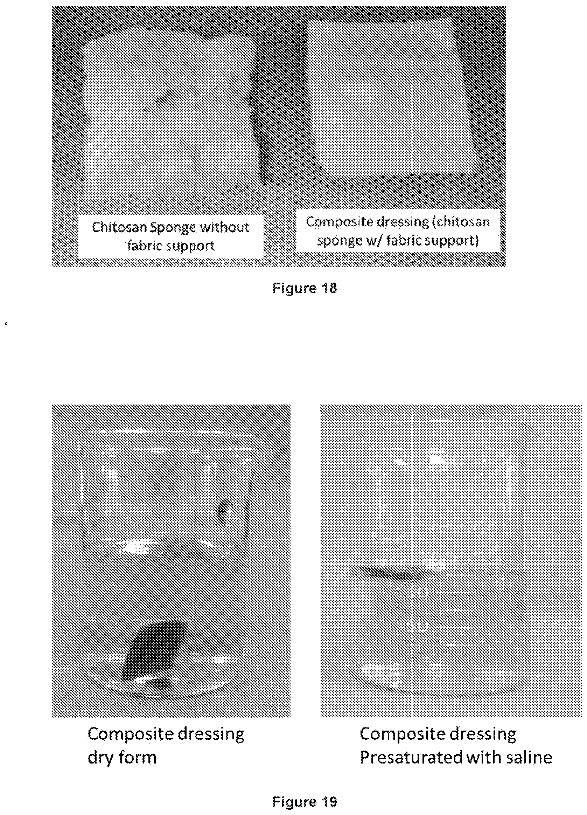

The composite dressing of the present invention is effective as hemostat both in dry as well as wet form. The application in wet form allows surgeons to use the composite dressing in same manner as the conventional gelatin foams. In order to soften the dressing, facilitate faster blood absorption and close contact with the wound site, the composite dressing is saturated with a hydration liquid followed by squeezing out the excess hydration liquid. Figure 18 depicts that composite dressings retain their texture and integrity even after they are completely soaked in the hydration liquid and squeezed, while the standalone porous matrices break into smaller pieced during squeezing. Due to the unique structure of the composite dressings of the present disclosure, said dressings can be wetted and squeezed without affecting their hemostatic properties. Figure 19 shows the *in vitro* hemostatic efficacy of composite dressings in both dry and wet form. As shown, in both cases the dressing absorbs the blood and immediately forms a blood clot, which remains locked within the dressing suggesting good hemostatic efficacy.

The hemostatic and bioadhesive properties of composite dressings can be further increased by using procoagulants as the hydration liquid. As described, the hydration liquid can be a procoagulant or combination selected from calcium chloride, calcium silicate nanoparticles, tranexamic acid, Faxtor X activators, Prothrombin activators, fibrin, Vitamin K, thrombin, platelet rich plasma etc. The hydration fluid can also be one or combinations of surgical sealants such as thrombm-fibrongen based sealants (Evicel), Gelatin-thrombin (Floseal), albumin, thrombin solution, or any other natural, semisynthetic or synthetic sealant formulation in liquid or semisolid form.

Adhesion force is maximum when the composite dressing is applied in dry form. However, the adhesion force of wet form is significantly improved by using either autologous platelet rich plasma from patient or recombinant procoagulant molecules for the wetting the composite dressing described above, allowing improved bioadhesion and efficient hemostasis.

The composite dressing, in dry or wet form, finds application to achieve hemostasis in different types of surgeries, where the bleeding site has irregular surface which may prevent application of conventional dressings impractical. Said surgeries include but are not limited to oozing from vascular suture lines, anastomosis site, spleen surgery, hepatic surgeries, nephrectomies and seroma formation after breast surgery. The composite dressing is also effective in controlling the bleeding in patients receiving high doses of anti-coagulant medications (e.g. heparin) or those having clotting disorders.

Accordingly, the present disclosure provides the above described composite dressing for use as a medicament. Said medicament is used for controlling bleeding; wherein the dressing is used as a hemostatic dressing; and wherein the bleeding is caused by surgical or traumatic wounds.

The present disclosure further relates to use of the above described composite dressing for controlling bleeding. Said bleeding is caused by surgical or traumatic wounds.

In an embodiment, but not according to the invention as claimed, embodiment, the present disclosure further provides a method of controlling or stopping bleeding, comprising contacting site of the bleeding with the above described composite dressing. The method comprises contacting the site of bleeding with the above described composite dressing in the required dimension, in dry or wet form. In an embodiment, in case of laparoscopic surgeries; the composite dressing is rolled into a tube or cylinder form and administered through the channel of laparoscope.

In a further embodiment, but not according to the invention as claimed, the present disclosure provides a method for controlling or stopping bleeding during surgery, said method comprising steps of:
a) aseptically soaking the composite dressing in a hydration liquid;
b) squeezing the dressing to remove the excess hydration liquid;
c) applying the treated composite dressing at site of the bleeding until hemostasis is achieved.

In an embodiment, the hydration liquid is selected from a group comprising water, saline, procoagulant material and natural, semisynthetic or synthetic sealants or any combinations thereof.

In an exemplary embodiment, the procoagulant material is selected from a group comprising calcium chloride, calcium silicate nanoparticles, tranexamic acid, Factor X activators, Prothrombin activators, fibrin, fibrinogen, Vitamin K, thrombin, aminocaproic acid and platelet rich plasma or any combination thereof; the sealant is surgical sealant selected from a group comprising thrombin-fibrinogen based sealants (Evicel), gelatin-thrombin (Floseal), albumin and thrombin solution or any combination thereof. The sealant is in liquid or semisolid form.

In an embodiment, in the method for controlling or stopping bleeding, the bleeding is from irregularly shaped wounds selected from a group comprising arterial anastomoses, hepatectomy, nephrectomy and seroma sites.

In a non-limiting embodiment of the disclosure, the bleeding is stopped by wrapping softened composite dressing around the site of bleeding. The dressing is softened by soaking in the hydration liquid and squeezed to remove the excess hydration liquid. The dressing remains integral even after being soaked in the hydration liquid and squeezed to remove the excess hydration liquid.

In a further embodiment, the method for controlling or stopping bleeding during surgical intervention further comprises removal of the dressing after bleeding is controlled, wherein said removal comprises step of soaking the dressing with excess hydration liquid selected from a group comprising water and saline or any combination thereof and removing it without dislodging the dressing.

The present disclosure therefore also provides a method of removal of the composite dressing from the site of application, but this method is not according to the invention as claimed. Chitosan-based hemostatic dressings are classified as non-absorbable hemostatic dressings and therefore they should be completely removed from the surgical wound sites once hemostasis is achieved. Complete removal, however, is a challenge in currently available forms. For example, chitosan hemostats are currently available in different forms such as: chitosan granules (Celox granules), chitosan granules impregnated on gauze (Celox Rapid/ Omni-stat), chitosan coated gauze (Chitogauze XR), non-woven chitosan gauze, and chitosan sponges or pads. A major problem with all granules and coated gauze forms is their removal from the surgical wound site. The granules adhere strongly the wound site and are not easily washed off. Chitosan is well known for its hemostatic activity. Several hemostatic products based on chitosan are available in market. Such dressings include chitosan sponges or pads, chitosan gauze dressing made using chitosan fibers and chitosan impregnated gauze dressings.

On the other hand, the composite hemostatic dressings of the present disclosure allow easy removal by their reversible bioadhesion properties. When the dressing is dry, the sponge/porous matrix side of the dressing strongly adheres to the wound site and provides mechanical seal against bleeding. The adhesion force of the dressings to the wound tissue is in range of 0.1 MPa to 1 MPa. This bioadhesive strength is comparatively higher in comparison to the prior art. The main reason for this is the cohesive structure and uniformly distributed pore size of sponge/porous matrix. The pores are present in a honeycomb like structure; which provides higher surface area to adhere with the wound surface. When the dressing is applied on the wound surface with manual compression it strongly adheres to the wound and creates a mechanical seal. A person skilled in the art would be aware of the quantum of pressure to be applied to the wound to keep the composite dressing in place to achieve hemostasis, taking into consideration severity of bleeding. In a non-limiting embodiment, the pressure is applied till hemostasis is achieved.

The dressing remains in position until hemostasis is achieved. Once the hemostasis is achieved, the dressing is removed by saturating it with saline, water or physiological buffers. The saturation process leads to reversal of bioadhesion and allows for easy removal of the dressing from the wound. Figure 20 shows the bioadhesion of composite dressings to a tissue surface and its removal after saturation with saline.

In an exemplary embodiment of the present disclosure, the dressing is completely removed, in a single piece, from the site of bleeding without any residues, leachable or particle release, after hemostasis. The dressings of the present disclosure therefore allow placement in a body cavity without a danger of leaving any residue. In an embodiment, removal of the dressing does not dislodge blood clot formed by the hemostasis.

In another embodiment, the present disclosure provides a kit comprising the composite dressing and hydration liquid as defined above, optionally along with an instruction manual. A person skilled in the art would be able to envisage more suitable applications of the composite dressing of the present disclosure. While the composite dressing of the present disclosure provides surprising effects and advantages over the conventional dressings, the said composite dressing is applicable for all purposes where a conventional dressing is capable of being employed. All such applications of the composite dressing herein, are within the scope of the present disclosure.

### EXAMPLES

The present disclosure is further described with reference to the following examples, which are only illustrative in nature and should not be construed to limit the scope of the present disclosure in any manner.

### Example 1: Preparation of 100% chitosan composite dressings

The two layers of the 100% chitosan composite dressing are a chitosan foam (sponge) and chitosan gauze (fabric). The preparation of composite dressing of the present disclosure involves following steps:

### Step i: Preparation of chitosan solution

A chitosan solution was prepared by dissolving chitosan powder or flakes of molecular weight about 400 kDa (ChitoClear, obtained from PRIMEX EHF, Oskarsgata 7, 580 Siglufjordur Iceland) in about 0.75% v/v aqueous acetic acid, such that the final chitosan concentration in the solution was about 2% w/v. Viscosity of the solution was maintained at >1000 cP by adjusting the chitosan and/or acid concentration.

### Step ii: Preparation of fabric substrate

### Step ii.a: Pre-treatment of ready-made chitosan gauze:

A chitosan gauze was selected as fabric substrate. As the chitosan gauze was made up of 100% chitosan, it had a tendency to dissolve in presence of dilute acidic solution such as the chitosan solution prepared in step 1. To prevent this, the chitosan gauze was pre-treated to limit its complete dissolution in the chitosan solution. For this, the chitosan gauze was completely dip-coated with a surfactant solution. Alternatively, one side of chitosan gauze was spray coated with the surfactant solution to improve its wettability and absorbency characteristics. Two different surfactants, polysorbate-20 or poloxamer P-407 at concentration of about 1% w/v and about 5% w/v were tested.

After the surfactant treatment, the chitosan gauze was exposed to elevated temperature of about 60°C for about 2 hours in a hot air oven, to reduce dissolution of the gauze in the biomaterial solution.

The obtained chitosan gauze was evaluated for its mechanical properties (tensile strength) and the wettability. The tensile strength was measured using a universal testing machine. The wettability properties were measured using a qualitative method. For this, chitosan gauze was cut into 2x2 cm pieces and the pieces were dropped in a beaker (250 mL capacity) filled with about 200 mL water and the time required for the pieces to completely sink in the water was noted down. Further, other parameters such as dry tensile strength, wet tensile strength and solubility in acetic acid were assessed.

As shown in Table 1, after the surfactant treatment followed by drying, the wetting properties of chitosan gauze improved significantly. Similarly, dry tensile strength and wet tensile strength of the chitosan gauze were found to improve significantly after surfactant treatment. Further, the surfactant treated chitosan gauze showed faster solubility in acetic acid, wherein when the chitosan gauze was treated with surfactant on one side, said side of the treated chitosan gauze showed faster solubility in acetic acid as compared to the rest of the gauze i.e. the untreated sides. Said observation implies that the surfactant treated chitosan gauze would provide increased susceptibility to and faster rate of dissolution when immersed in solutions of biomaterial intended to form the porous matrix of the composite dressing, wherein said solutions are prepared in organic acid solvents. This would fasten the process of preparation of the composite dressing. Further, the reduced wetting time would confer improved absorption properties to the surfactant treated chitosan gauze.

**Table 1: Properties of surfactant treated fabric substrate**

| **Fabric type** | **Dry Tensile strength (Avg)** | **Wet Tensile strength (Avg)** | **Wetting time** | **Solubility in 0.75% acetic acid** |
|---|---|---|---|---|
| Untreated (no surfactant treatment) | 1.66 ± 0.35 MPa | 0.4 ± 0.1 MPa | >90 sec | Complete dissolution in 2 h |
| Dip coated | | | | Complete dissolution in 30 min |
| a. 1% Polysorbate 20 | 3.12 ± 0.11 | 1.69 ± 0.74 | < 5 sec | |
| b. 5% Polysorbate 20 | MPa | MPa | < 5 sec | |
| c. 1% Poloxamer | 3.61 ± 0.45 | 1.85 ± 0.87 | < 5 sec | |
| P407 | MPa | MPa | < 5 sec | |
| d. 5% Poloxamer | 3.39 ± 0.28 | 1.74 ± 0.68 | | |
| P407 | MPa | MPa | | |
| | 3.35 ± 0.51 | 1.76 ± 0.71 | | |
| | MPa | MPa | | |
| Spray coated on one side | 2.67 ± 0.37 | 1.27 ± 0.27 | ~15 sec | Surfactant coated side dissolves in 30 min, uncoated surface remain stable for 120 min |
| a. 1% Polysorbate 20 | MPa | MPa | ~15 sec | |
| b. 5% Polysorbate 20 | 2.42 ± 0.32 | 1.42 ± 0.42 | - 15 sec | |
| c. 1% Poloxamer | MPa | MPa | ~ 15 sec | |
| P407 | 2.34 ± 0.58 | 1.34 ± 0.18 | | |
| d. 5% Poloxamer | MPa | MPa | | |
| P407 | 2.26 ± 0.33 | 1.26 ± 0.23 | | |
| | MPa | MPa | | |

### Step ii.b: Preparation of fabric substrate with mixed fiber:

A special fabric was obtained by mixing fibers of variable wetting and swelling properties. To achieve this, crosslinked chitosan fibers and acid-treated (activated) chitosan fibers were prepared separately as mentioned below.

First, about 10-20% w/v chitosan fibers were suspended in water containing about 1% w/v glutaraldehyde for about 24 hours. After the glutaraldehyde treatment, the fibers were washed with water and dried at about 60 °C. These crosslinked fibers show better stability in water and aqueous solution of acetic acid.

Acid-treated chitosan fibers were prepared separately by suspending about 10-20% w/v chitosan fibers in ethanol and adding about 1% v/v acetic acid to it. The suspension was mixed for 1 hour at room temperature. The acid treated fibers were recovered from suspension by filtration and dried at about 60°C for about 4 hours.

About 10% w/w-50% w/w of crosslinked fibers were then mixed with about 50% w/w-90% w/w acid-treated chitosan fibers and processed through a needle punching apparatus to obtain a non-woven fabric.

The obtained fabric was evaluated for its mechanical properties (tensile strength), wettability and solubility in organic acid. Table 2 below shows the properties of fabrics obtained using this method.

It was observed that the mixed fiber fabric substrate showed better wettability i.e. reduced wetting time and at the same time had improved stability in aqueous solution of acetic acid as compared to unprocessed chitosan gauze (Table 1). Said observation implies that the mixed substrate would provide increased susceptibility to being wetted when immersed in solutions of biomaterial intended to form the porous matrix of the composite dressing, wherein said solutions are prepared in organic acid solvents. This would fasten the process of preparation of the composite dressing. To form the composite dressing of the present disclosure, it is necessary that the fabric substrate has good wettability in the biomaterial solution but at the same time does not undergo complete dissolution. A certain percent of undissolved fibers is necessary to provide suitable mechanical properties (under wet conditions) to the composite dressings. As shown in Table 2, the mixed fiber fabric substrate became more stable with increasing amount of crosslinked fibers in the fabric. The mixed fiber fabric substrate composed of 50% crosslinked fibers and 50% acidified fibers, showed good wettability and yet remained stable in the acetic acid solution for 6 hours. Further, the reduced wetting time would also confer improved absorption properties to the mixed fabric substrate.

**Table 2: Properties of the mixed fabric substrate**

| **Composition of fabric** | **Dry Tensile strength (Avg)** | **Wet Tensile strength (Avg)** | **Wetting time** | **Solubility in 0.75% acetic acid** |
|---|---|---|---|---|
| 10 % crosslinked + 90% acidified | 2.66 ± 0.35 MPa | 0.21 ± 0.1 MPa | < 5 sec | Dissolves instantly |
| 30 % crosslinked + 70 % acidified | 3.26 ± 0.63 MPa | 0.72 ± 0.32 MPa | < 5 sec | Dissolves in 2 hours |
| 50% crosslinked + 50% acidified | 3.98 ± 0.59 MPa | 1.76 ± 0.53 MPa | < 5 sec | Stable for 6 hours |

### Step iii: Assembly and lyophilization

As outlined in Figure 2, the chitosan solution (5) obtained in step 1 was poured in molds (4) of predefined dimensions. The chitosan gauze/surfactant treated chitosan gauze/mixed fabric chitosan gauze (2) was carefully lined on top of the chitosan solution. During this step, the chitosan fibers of gauze were wetted with the chitosan solution.

The viscosity of chitosan solution was maintained at >1000 cP to control the extent of interaction between the chitosan fibers and chitosan solution. All the fabric substrate samples prepared in Step (ii), remained on surface of chitosan solution having this viscosity. In all cases about 50% of chitosan fibers of lined chitosan gauze were wetted by the chitosan solution.

The complete assembly was then placed in a lyophilizer and freeze-dried at predefined lyophilization cycle, wherein first the temperature of assembly was gradually reduced from about 25 °C to -30 °C in about 8 hours, then the frozen assembly was exposed to a controlled vacuum at about -30°C for about 36 hours. During this step, the pressure in the lyophilizer chamber was gradually reduced from about 250 mTorr - 70 mTorr in about 12 hours and then maintained for about 12 hours and slowly increased to about 250 mTorr in about 12 hours. Finally, a secondary drying was carried out and the temperature of assembly was increased from about 15°C - 35°C in about 6 hours.

During lyophilization, the chitosan solution was converted into a chitosan sponge in which the chitosan fibers were embedded.

### Example 2: Characterization of the 100% chitosan composite dressing

The chitosan based composite dressing obtained using aforesaid method comprising fabric substrate pre-treated by dip-coating with 1% Polysorbate 20 was characterized to evaluate their internal structure, fluid absorbency, mechanical properties and hemostatic ability.

### a) Characterization of internal structure of composite dressings

The internal structure of the composite dressing was observed using the scanning electron microscopy (SEM). The results are shown in Figure 7a, wherein the SEM micrographs clearly showed the distinct porous matrix bonded to a chitosan gauze layer. The pore size of the chitosan sponge was between about 10 µm -300 µm and the average pore size was about 100 µm. The wall thickness of the porous matrix was at least 1 µm.

The gauze layer of the composite dressing appeared intact, with its fibers embedded within the porous matrix of chitosan. The thickness of the bonding was ranging from about 0.1 mm to 0.2 mm (Figure 7b).

### b) Fluid absorbency and swelling behavior

Fluid absorbency is an important property of the composite dressing. To evaluate this, a 2×2cm piece of composite dressing was placed in about 50 mL of water and allowed to swell for about 1 hour. The composite dressing showed fluid absorption of at-least about 20 times its weight, more specifically between about 30 to 60 times. Similarly, the porous matrix (chitosan sponge) of the dressing swelled at-least about 1.5 times its original size (Figure 8). Conventional composite dressings prepared using adhesive layer tend to separate after being placed in excess amount of fluids such as water (Figure 12). Similarly, composite dressings prepared using stitching damages the chitosan sponge and breaks apart after swelling (Figure 12). In contrast, the composite dressing of the present remains intact and works as a unit even after absorbing large quantities of fluids.

### c) Mechanical properties of the composite dressings

The composite dressing exhibited excellent mechanical properties for easier handling and safety during application. Good mechanical properties are necessary to ensure that the dressing does not disintegrate or break apart within a body cavity during the application. The mechanical properties such as peel strength, tensile strength and elongation at break under dry and wet conditions were evaluated using a universal testing machine (UTM). For these tests, composite dressing samples were specifically prepared and mounted on the UTM as shown in Figure 9. For wet strength measurement, the samples were saturated with water and tested using UTM without squeezing out the excess water.

The peel strength study indicated that the bonding between the porous matrix and the fabric support was considerably higher than the individual tensile strengths of fabric support or porous matrix. Figure 9 depicts the images of samples used for estimation of bonding strength of fabric support (2) on the porous matrix (1). As shown, during the experiment, the bonding between the layers remained intact in both dry and wet conditions. However, the fabric support or porous matrix broke horizontally under dry or wet conditions, respectively. Said bonding strength is the result of partial embedding and dissolution of fabric support in the porous matrix resulting in very strong bonding between the two layers.

These results are presented in the table below.

**Table 3: Dry strength and wet strength of the composite dressing of the present disclosure**

| Test | Dry strength | Wet strength |
|---|---|---|
| Tensile strength | 3.09 ± 0.1 MPa | 0.7 ± 0.19 MPa |
| Elongation at break | 10% ± 5% | 10% ± 5% |

### d) In vitro hemostasis properties and comparison with products of the prior art

*In vitro* hemostatic activity of the composite dressing was evaluated using the whole blood clotting assay and percentage hemolysis test. The results indicated that the composite dressing achieved complete hemostasis in under about 3 minutes and formed a strong blood clot that did not release any free erythrocytes when placed in excess water (Figure 10).

### Example 3: Analysis of the effect of a combination of fabric substrate and porous matrix on characteristics of the dressing

Preparation and characterization of chitosan sponges without a fabric support:
A chitosan solution was prepared by dissolving chitosan powder (2% w/v) in dilute acetic acid solution (0.75% w/v). The chitosan solution was then poured into stainless steel moulds and lyophilized as described in Example 1.

The chitosan sponges without the fabric substrate were characterized for their dry and wet tensile strength, absorbency and wet integrity testing under simulated clinical use, and compared with corresponding parameters of the composite dressing of the present disclosure (table 4).

The dressings (chitosan sponges with and without fabric substrate) of dimension 5x5 cm were immersed in 100 ml of saline for 5 minutes and allowed to swell completely. The excess saline was then squeezed out by pressing with fingers. The photographs of these dressings are depicted in Figure 11. Figures A1/A2 & B1/B2 represent the chitosan sponges without fabric support and represent chitosan composite dressings, respectively. Top panel in said figure depicts the dry dressings, while the bottom panel shows dressings being wetted in saline and squeezed to remove excess fluid. The composite dressings retained their smooth texture and integrity (B2), while the standalone chitosan sponge broke into smaller pieces (A2) -

**Table 4: Comparison between chitosan sponge mechanical properties vs chitosan composite dressing**

| **Properties** | **Chitosan sponge** | **Chitosan composite dressing** |
|---|---|---|
| Dry tensile strength | 1.8 ± 0.2 MPa | 3.09 ± 0.1 MPa |
| Wet tensile strength | 0.03 ± 0.01 MPa | 0.7 ± 0.19 MPa |
| Flexibility | Stiff, breaks when bent | Flexible |
| Maximum bend angle | 90 degree (break when bent >90 degree) | 180 degree (doesn't break) |
| Absorbency in water | 39 ± 15 times the weight of sponge | 42 ± 15 times the weight of dressing |
| Absorbency in blood | 20 ± 5 times the weight of sponge | 21 ± 7 times the weight of dressing |

### Example 4: Composite dressings made using stitching or adhesive layer

To obtain stitched composite dressings, the chitosan sponge was stitched on the chitosan fabric using a sewing machine (Figure 12, C1/C2).

Another composite dressing (Figure 12, D1/D2) was prepared by using a double-sided adhesive tape (3M) to hold chitosan fabric and sponge together.

The stitching damaged the porous structure of the sponge and resulted in breaking of sponge at the stitching line. The adhesive was wash proof, yet the sponge layer separated from the adhesive layer due to disproportionate swelling of sponge. Chitosan sponges tend to swell at least 1.5 times their size in water. The composite dressing prepared by application of adhesive or stitching of the fabric support restricts this swelling, which resulted in either separation of layers of breaking of sponge (Figure 12, C2 and D2). On the other hand, composite dressings of the present disclosure allow chitosan sponge to swell without separation as the fibers of fabric support are deeply embedded in the chitosan sponge.

### Example 5: Composite dressing of starch and chitosan

To obtain the composite dressing of starch and chitosan, starch was used as porous matrix and chitosan gauze was used as fabric support. About 3g of pregelatinized starch (Starch 1500; gift sample from Colorcon, Inc. 420 Moyer Blvd, West Point, PA USA.19486) was suspended in about 100mL of deionized water and the suspension was autoclaved. The autoclaving process resulted in a viscous solution of starch, which was used as a starting solution to prepare the porous matrix of the composite dressing. The starch solution was then poured into metal molds and the chitosan gauze was placed on to it and a second layer of the starch solution was added on top of the chitosan gauze. The composite dressing was obtained by the lyophilization as described in Example 1, Step iii. The resulting composite dressing had a sandwich type configuration as shown in Figure 4. It contained a layer of chitosan gauze sandwiched between two layers of starch porous matrix.

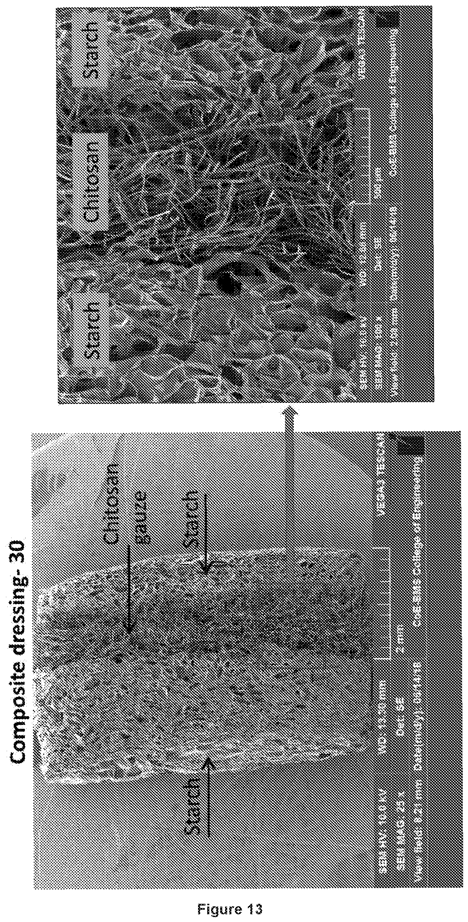

The resulting starch-chitosan composite dressing was observed using Scanning Electron Microscopy (SEM). The images showed distinct porous matrices of starch attached to the fabric layer of chitosan (Figure 13). It was noteworthy that at least 50% of the fibers of chitosan gauze remained undissolved and retain their distinct structure despite being sandwiched between the aqueous starch solution.

While starch-based sponges have poor mechanical properties, their combination with chitosan gauze as fabric substrate to form composite dressing yield dressing having good mechanical integrity. The results of mechanical testing of these dressings showed that the dry tensile strength was about 3.98 ± 1.2 MPa and wet tensile strength was about 2.3 ± 0.92 MPa. These values are comparable to 100% chitosan composite dressings. The formation of composite dressings also improved the disintegration behavior of starch-based sponges. The starch-based sponges generally disintegrated easily after adding to aqueous media, whereas the composite dressing comprising the starch-based sponges in combination with chitosan gauze as fabric substrate remained stable for at-least about 24 hours in aqueous media. The improved mechanical properties of the starch-chitosan composite dressing make it fit for applications such as hemostatic dressings in surgical bleeding and internal hemostasis.

### Example 6: Composite dressing of sodium alginate and chitosan

The composite dressings containing sodium alginate were prepared as explained in Example 1. Except for the fact, that sodium alginate solution (1% (w/v)) was used instead of chitosan solution for the porous matrix. The alginate solution was poured in the moulds of predefined sizes and the chitosan fabric was lined on top of the alginate solution.

In a variation of the experiment, the chitosan fabric was of a mixed fabric type (50% acidified and 50% crosslinked as shown in Table 2). The cationic charge of acidified chitosan fibers resulted in the cross linking of anionic alginate solution at the interface and increased its tensile strength.

In another variant, the chitosan fabric support was pretreated with calcium chloride solution, which also led to crosslinking of alginate solution and provided composite dressings with higher tensile strength and greater bending angle.

The flexibility and tensile strength of the three variants were assessed and the following observations were made -

**Table 5: Properties of composite alginate dressings**

| **No** | **Sponge material** | **Fabric support** | **Results** |
|---|---|---|---|
| 1. | Sodium alginate | No | Brittle sponge, disintegrates instantly in water. |
| 2. | Sodium alginate | Chitosan fabric (50% acidified + 50% crosslinked) | Flexible sponge, bend angle of minimum 90 degrees. |
| | | | Remains stable in water |
| | | | Tensile strength comparable to fabric support. 1.89 ± 0.22 MPa |
| 3. | Sodium alginate | Chitosan fabric, pretreated with calcium chloride solution | Slightly rigid sponge, bend angle 90 degree. |
| | | | Remains stable in water. |
| | | | Higher tensile strength. |
| | | | 2.69 ± 0.17 MPa |

### Example 7: Composite dressing of Polyethylene Glycol (PEG) and Chitosan

The composite dressings were successfully prepared by lyophilizing PEGs of 5000Da, 10000Da and 35000Da with a mixed type chitosan fabric support. For this, PEG solutions were poured in the moulds and lined with the fabric support (mixed type fabric containing 50% crosslinked and 50% acidified chitosan fibers). The acidified chitosan fibers absorbed the PEG solution and swell at the interface. After lyophilization, the PEG was entangled in the mesh of chitosan fibers. The resulting dressings had good mechanical properties and did not disintegrate in water instantly.

Similar composite dressings were also prepared by lyophilizing polyethylene oxide (PEO) which are basically PEGs having molecular weight above 100000 Da. When lyophilized, PEO alone gave very rigid sponges which broke when bent (bend angle < 90 degree). However, the composite dressings made using chitosan fabric as support resulted in flexible sponges with good mechanical properties.

**Table 6: Properties of PEG - chitosan composite dressings**

| **No** | **Sponge material** | **Fabric support** | **Results** |
|---|---|---|---|
| 1. | PEG 5000 (MW 5000 Da) | No | Brittle cake, lack mechanical strength to handle |
| 2. | PEG 10000 (MW 10000 Da) | No | Brittle cake, lack mechanical strength to handle |
| 3. | PEG 35000 (MW 35000 Da) | No | Brittle cake, lack mechanical strength to handle |
| 4. | PEO (MW > 100000 Da) | No | Rigid cake, bend angle < 90 degree disintegrates in water instantly |
| 5 | PEG 5000 | Chitosan fabric | Soft flexible sponge, remains stable in water, Bend angle 180 degrees |
| 6 | PEG 10000 | Chitosan fabric | Soft flexible sponge, remains stable in water, Bend angle 180 degrees |
| 7 | PEG 35000 | Chitosan fabric | Soft flexible sponge, remains stable in water, Bend angle 180 degrees |
| 8 | PEO (MW > 100000 Da) | Chitosan fabric | Soft flexible sponge, remains stable in water, Bend angle 180 degrees |

### Example 8: Composite dressing of Hyaluronic acid (HA) and chitosan fabric

Conventionally prepared HA sponges dissolve instantly in water if prepared without a crosslinking agent. Stable HA sponges of the present disclosure were prepared without addition of any crosslinking agent. The crosslinking was achieved by the chitosan fibers.

The composite dressing was prepared as explained in Example 1. The HA solution was poured into moulds and lined with a mixed type chitosan fabric (50% acidified with 50% crosslinked fabric) as shown in Table 2. The acidified chitosan fibers swelled in the HA solution while the crosslinked chitosan fibers provided the strength to the resulting sponges. The resulting sponges had a tensile strength of at least 1.5 MPa.

### Example 9: Composite dressings of carboxymethyl cellulose (CMC) and chitosan

Two types of composite dressings containing carboxymethyl cellulose (CMC) and chitosan were prepared as explained below;
a. Carboxymethyl cellulose sponge attached to chitosan fabric: To obtain this, 2% w/v CMC was dissolved in water. The dissolution is increased by slightly warming the solution. The resulting viscous solution was poured in moulds and lined with chitosan fabric. The composite dressings were obtained by lyophilization as discussed in Example 1. The resulting sponges had good mechanical properties (dry tensile strength of at least 1.89 MPa and wet tensile strength of at least 0.2 MPa) with bending angle close to 180 degrees.
b. Chitosan sponge attached carboxymethyl cellulose fabric: To obtain this, chitosan solution was prepared as discussed in Example 1, the chitosan solution was poured in a mould and lined with a nonwoven CMC fabric. The CMC fibers have gelling properties and absorb water to form a thick gel. However, in case of chitosan solution, some portion of CMC swelled and precipitated at the interface due to acidic pH of chitosan solution. This resulted in malleable sponges with distinct CMC fabric side and chitosan sponge side.

### Example 10: Composite dressing of rayon polyester and chitosan fabric

A chitosan composite dressing was fabricated with an untreated rayon-polyester nonwoven fabric substrate wherein the ratio of rayon:poly ester was 70:30. Chitosan solution was poured into a mold and abovementioned fabric substrate was placed on top of the chitosan solution, before lyophilization. The procedure of Example 1 was followed to prepare the composite fabric. The thickness of the composite was about 3mm after lyophilization. The said composite dressing had physical properties provided in Table 7.

In a variant of the experiment, the fabric substrate can also be placed as the bottom layer or both on top and at the bottom to give more strength to the composite dressing.

The composite dressing was characterized as follows -

**Table 7: Properties of rayon polyester and chitosan dressing**

| Absorbency | 40 times the weight of dressing |
|---|---|
| Dry tensile strength | 5 ± 1.4 MPa |
| Wet tensile strength | 2.1 ± 0.6 MPa |
| pH | 5.6 ± 0.6 |
| Moisture content | 23% ± 3.8 |

### Example 11: Composite dressings with X-ray detectable element

Two variants of composite dressing of Example 10 with X-ray detectable element sandwiched between the fabric support and porous matrix were prepared and radiopacity of the composite dressings was measured. Two different designs of composite dressings were made to for the radiopacity testing - First design with a single radiopaque element in horizontal line and second design with a radiopaque thread placed in a grid pattern. Placing the X-ray detectable thread in a grid pattern also helped in reinforcing the chitosan sponge to improve its strength.

The radiopacity testing was performed in accordance with ASTM F640-12 (Standard test methods for determining radiopacity for medical use). The test specimens included a composite dressing with a single x-ray detectable thread, a composite dressing with a grid pattern x-ray detectable threads, and standard gauze product with an x-ray detectable element. X ray images were taken at a voltage of 57 KV, current of 200 mA, and exposure time of 20 mAs (milliampere seconds).

The radiopacity of all test specimens and user defined standard was evaluated qualitatively as per ASTM standard in terms of image background with and without use of body mimic. As shown in Figure 15, both test samples and a control sample were detectable under x-ray. To confirm the radiopacity of dressings inside the body, the dressings were placed beneath a piece of meat and their x-ray images were acquired. As shown in Figure 16, dressings of the present disclosure provide sufficient radiopacity.

### Example 12: Hemostatic efficacy of composite dressings in anticoagulated population or those with clotting disorders

To evaluate the efficacy of composite dressing in anticoagulated populations, a preclinical study was conducted in heparinized sheep. For this, both superficial femoral arteries were catheterized with 8F sheaths for 15 minutes. The composite dressing was held on the right femoral artery puncture site and catheter was slowly removed. The left femoral artery puncture site was used as a control treated with standard cotton gauze dressing. Manual compression was applied on both sites and time to hemostasis was recorded for both groups. Hemostasis time in the composite dressing group was significantly less than the control. This animal study demonstrated the composite dressings were able to control bleeding in an anticoagulated model.

The mean hemostasis time was 3.5 minutes in sheep treated with the composite dressings, whereas the mean hemostasis time in control group was 15 minutes. The mean blood loss was significantly lower (5.6 ml) in case of composite dressing in comparison to the control group (10.8 ml). Additionally, no hematoma, rebleeding was observed in any sheep treated with the composite dressings. Overall, the results showed that the composite dressings are effective in controlling bleeding in anti-coagulated population.

### Example 13: Efficacy of composite hemostatic dressings in partial nephrectomy patients

The hemostatic efficacy of composite hemostatic dressings was evaluated in an observation study in a patient undergoing partial neprectomy surgery. The patient had a prior history of similar surgery and a tumor developed on the right kidney. The composite dressing was applied after the excision of the tumor from the right kidney and pressure was given for 5 minutes. The surgeon's feedback was recorded as in Table 8.

**Table 8: Results of application in partial nephrectomy patients**

| **Parameters** | **Patient outcome** |
|---|---|
| Time to Hemostasis | 5 minutes |
| Ease of application | Excellent |
| Ease of removal | Fair |
| Adherence to wound | Good |
| Conformability of dressing | Excellent |
| Patient comfort | Excellent |

### Example 14: Efficacy of composite hemostatic dressing during cardioverter defibrillator implantation

An implantable cardioverter-defibrillator is a device which is able to perform cardioversion defibrillation and pacing of the heart once implanted inside the body. It is, therefore, capable of correcting most life-threatening cardiac arrhythmias. The dressing was used at the implantation site. The time taken to achieve hemostasis was 5 minutes post application of dressing to control device related bleeding. The dressing was removed by irrigating with saline, the operative site showed no signs of re-bleeding, swelling, vascular complication or allergy after removal of dressing. Overall, the composite hemostatic dressing was found to be an excellent hemostat to control bleeding intraoperatively as shown in the case. The surgeon's feedback was recorded as in Table 9.

**Table 9: Results of application during cardioverter defibrillator implantation**

| **Parameters** | **Patient outcome** |
|---|---|
| Time to Hemostasis | 5 minutes |
| Ease of application | Excellent |
| Ease of removal | Excellent |
| Adherence to wound | Excellent |
| Conformability of dressing | Excellent |
| Patient comfort | Excellent |

### Example 15: Efficacy of composite hemostatic dressing during pacemaker implantation

The hemostatic efficacy of composite hemostatic dressings was evaluated in an observation study in a patient undergoing a surgery for implantation of a pacemaker. The patient was suffering from diabetes and hypertension. During the procedure, a pocket was created to implant the pacemaker. The implantation site caused profuse bleeding from the pocket and composite hemostatic dressing was then used to control the bleeding. The bleeding was stopped in 3 minutes. The surgeon's feedback was recorded as in Table 10.

**Table 10: Results of application during cardioverter pacemaker implantation**

| **Parameters** | **Patient outcome** |
|---|---|
| Time to Hemostasis | 5 minutes |
| Ease of application | Excellent |
| Ease of removal | Good |
| Adherence to wound | Excellent |
| Conformability of dressing | Excellent |
| Patient comfort | Excellent |

### Example 16: Efficacy of the composite hemostatic dressing during Aorta-bifemoral bypass surgery

The hemostatic efficacy of composite hemostatic dressings was evaluated in an observation study in a patient undergoing the aorta-bifemoral bypass surgery. Bypass surgery is performed to clear the blockage in the abdominal aorta. This treatment uses a graft to reroute blood around a blocked artery in the abdomen or thigh. Bleeding and blood clots are the most common and high risk associated with this surgery. Therefore, bleeding control becomes an essential part of the procedure keeping in mind the time taken and the doctor compliance. The composite dressing offered quick hemostasis preventing blood loss and easy and complete removal of the dressing from the wound site. The surgeon's feedback was recorded as in Table 11.

**Table 11: Results of application during Aorta-bifemoral bypass surgery**

| **Parameters** | **Patient outcome** |
|---|---|
| Time to Hemostasis | 4 minutes |
| Ease of application | Excellent |
| Ease of removal | Good |
| Adherence to wound | Excellent |
| Conformability of dressing | Excellent |
| Patient comfort | Excellent |

### Example 17: Efficacy of composite hemostatic dressing during orthopedic surgery

The hemostatic efficacy of composite hemostatic dressings was evaluated in an observation study in a patient undergoing an orthopedic surgery. The patient encountered distal tibia fracture caused by sharp penetration trauma. Fractures of the distal third tibia are unique in that the bone is subcutaneous with depleted muscular cover; the consequent decreased vascularity leads to complications like delayed bone union, wound complications such as dehiscence and infection. These fractures can be managed with various techniques.

The composite dressing of the present disclosure was used to stop the bleeding from the fracture site. The hemostasis time was 1 minute 20 seconds, which was significantly shorter than the conventional method of applying manual compression with cotton gauze, which usually requires around 20 minutes. The patient outcome was recorded as in Table 12.

**Table 12: Results of application during orthopedic surgery**

| **Parameters** | **Patient outcome** |
|---|---|
| Time to Hemostasis | 1 minute 20 secconds |
| Ease of application | Good |
| Ease of removal | Good |
| Adherence to wound | Good |
| Conformability of dressing | Good |
| Patient comfort | Excellent |

### Example 18: Composite dressings comprising pre-treated fabric substrates

The rayon-polyester fabric support described in Example 10 was first treated with glycerol, Polyethylene-glycol (PEG) 10000 and PEG 35000 to improve its flexibility and wettability. As a result of the improved surface properties of the fabric substrate, the composite dressings made using these substrates had a smoother surface, uniform pore size, better flexibility and better wet strength as compared to chitosan sponges made without the fabric substrate.

The improved wet strength and stability of the composite dressings was due to the displacement of bound acetic acid from chitosan molecules during the lyophilization process. The displacement of bound acetic acid led to higher evaporation of acetic acid during the lyophilization cycle. This was evident from the acetic acid contents of the chitosan sponges and composite dressings. The acetic acid content of final sponges and composite dressings was quantified using the titration again 0.01 N NaOH solution. The results are presented in Table 13.

**Table 13: Properties of composite dressings comprising pre-treated fabric substrates**

| **Dressing type** | **Fabric substrate** | **Fabric treatment** | **Acetic acid content in dressing** | **Flexibility (ability to roll the dressing into a cylinder)** | **Stability in water** |
|---|---|---|---|---|---|
| Chitosan Sponge | No | None | 26 ± 6.42 % | Cannot be rolled | Disintegrates in 6 hours |
| Composite dressing | polyester-rayon gauze | No treatment | 19 ± 4.65 % | Can be rolled into a tube of 5-8 mm dia | Disintegrates in 12 hours |
| Composite dressing | polyester-rayon gauze | Glycerol | 18 ± 4.82 % | Can be rolled into a tube of 5-8 mm dia | Disintegrates in 24 hours |
| Composite dressing | polyester-rayon gauze | PEG 10000 | 14 ± 5.23 % | Can be rolled into a tube of 3-5 mm dia | Stable for >7 days |
| Composite dressing | polyester-rayon gauze | PEG 35000 | 21 ± 1.23 % | Can be rolled into a tube of 3-5 mm dia | Stable for >7 days |

### Example 19: Compressed composite dressings

The flexibility of the composite dressings was further improved by compressing the composite dressing. The rayon-polyester fabric support and chitosan sponge based composite dressing of Example 10 was subjected to compression using a roller mill.

The final thickness of composite dressings was about 0.2 mm. The compressed dressings were flexible enough to be rolled into a tube or cylinder shape of 2-3 mm diameter.

The internal structure of the compressed composite dressings was evaluated using scanning electron microscopy. The fabric support was embedded in the chitosan sponge and depth of the interaction was at-least 0.1 mm. The sponge was found to have uniformly distributed porous structure without any crystalline or granulation areas. The pore size was in a range of 100 - 300 micron and the wall thickness of the porous matrix was at least 1 µm.

### Example 20: Novel method of using chitosan hemostatic dressings during surgical intervention

A kit was assembled containing packaged composite dressing of the present disclosure along with a hydration liquid such as saline in a container. The kit was put in application for the below working examples-

### 20.1: Dressing integrity after soaking in fluids and squeezing

The composite dressings of the present disclosure are highly flexible and have good wet strength. The method of using the dressing during surgical intervention involves, saturating the composite dressing with hydration liquid and squeezing out excess hydration liquid.

To analyze the efficacy, the composite dressing of Example 10 from the kit was saturated with saline in said kit and then the excess saline was squeezed out. Said process softened the dressing and allowed for faster blood absorption and close contact with the wound site As shown in Figure 18, the composite dressing retained its texture and integrity even after it was completely soaked in saline and squeezed, while the standalone chitosan sponges broke into smaller pieced during squeezing.

### 20.2 Hemostatic efficacy of composite dressings after soaking and squeezing in saline

To evaluate the hemostatic efficacy, dry or wet pieces of said dressing (5×5 cm) were placed in a beaker and 1 ml of blood was added to each piece. After allowing to form a blood clot for 1-minute, excess water was added to the dressings. A poor blood clot/ hemostasis was indicated by the release of un-clotted blood cells in the water and washing off the blood clot from the gauze. Figure 19, shows the *in vitro* hemostatic efficacy of composite dressings in both dry and wet form. As shown, in both cases the dressing absorbed the blood and immediately formed a blood clot, which remained locked within the dressing suggesting good hemostatic efficacy.

### 20.3 Presaturation of composite dressings with pro-coagulants, biomolecules and surgical agents

The hemostatic and bioadhesive properties of composite dressings can be further increased by using procoagulants as the hydration liquid. To check the benefits of using procoagulants with composite dressing, and *in vitro* hemostasis and bioadhesion test was performed. For the blood clotting test, the composite dressing was cut into 1×1 cm piece (weighing 30 mg), soaked in a procoagulant solution (2mM calcium chloride solution) and squeezed to remove excess fluid. The piece was then placed in a test-tube and 1 ml blood was added to it. The tubes were incubated at 37°C. The clot formation was checked by inverting the test-tubes and the whole blood clotting time was defined as the time when no free-flowing blood was observed in the tube.

The bioadhesion force was measured by applying the dressings containing procoagulant to a piece of meat. The bioadhesion force was quantified as the force required to separate the dressing from the meat piece.

The results are presented in Table 14. The composite dressing was used as the base hemostatic dressing. The adhesion force was maximum in when the composite dressing was applied in dry form. However, the adhesion force was significantly improved by using either autologous platelet rich plasma from patient or recombinant procoagulant molecules for improved bioadhesion and efficient hemostasis.

The method described above can be used to achieve hemostasis different types surgeries, where the bleeding site has irregular surface which may prevent application of conventional dressings impractical.

**Table 14: Effect of hydration liquid**

| **No.** | **Hydration liquid** | **Whole blood clotting time** | **Bioadhesion force** |
|---|---|---|---|
| 1. | Dry composite dressing alone (no hydration) | 30 seconds | 0.76 ± 0.28 MPa |
| 2. | Saline | 60 seconds | 0.14 ± 0.09 MPa |
| 3. | Calcium chloride (5 mM) | 30 seconds | 0.18 ± 0.06 MPa |
| 4. | Tranexamic acid | 50 seconds | 0.17 ± 0.08 MPa |
| 5. | Platelet rich plasma | 30 seconds | 0.29 ± 0.16 MPa |
| 6 | Thrombin solution | 15 seconds | 0. 3 5 ± 0.11 MPa |
| 7. | Floseal (Gelatin matrix with thrombin) | 15 seconds | 0.38 ± 0. 09 MPa |
| 8. | Tisseel (sealer protein solution with thrombin solution) | 15 seconds | 0.36 ± 0.12 MPa |
| 9. | Bioglue (Albumin with crosslinking agents) | 15 seconds | 0.45 ± 0.17 MPa |

### Example 21: Reversible bioadhesive properties of the composite hemostatic dressings

When the dressing is applied on the wound surface with manual compression it strongly adheres to the wound and creates a mechanical seal. The seal remains in position until hemostasis is achieved.

To remove the dressing, it was saturated with saline. The saturation process leads to reversal of bioadhesion and allows for easy removal of the dressing from the wound surface without dislodging the blood clot. The strong bioadhesion between dressing and wound surface is depicted in Figure 20.

## Claims

1. A removable composite dressing comprising at least one layer of porous matrix composed of biomaterial and at least one layer of fabric support, wherein the fabric support is at least partially embedded and dissolved in the porous matrix; and wherein at least 50% of fibers from the fabric support remain undissolved within or outside the porous matrix.

2. The composite dressing as claimed in claim 1, wherein the porous matrix and the fabric support are both composed of biomaterial, and wherein the porous matrix and the fabric support are both composed of same or different biomaterial;
wherein the biomaterial is selected from a group comprising chitosan, alginates, starch, cellulose, gelatin, collagen, Polyethylene Glycol (PEG), Polyvinyl Alcohol (PVA), polyurethanes, hyaluronic acid, poly-lysine, poly-glutamic acid, poly(ethylene oxide) (PEO), poly(propylene oxide) (PPO), poly(acrylic acid), poly(methacrylic acid) and their derivatives or any combination thereof;
wherein the derivative of chitosan is selected from a group comprising chitosan-PEG and hydrophobically modified chitosan; wherein the derivative of starch is selected from a group comprising pregelatinized starch, carboxymethyl starch and sodium starch glycolate; and wherein the derivatives of cellulose is selected from a group comprising oxidized cellulose, methyl cellulose and carboxymethyl cellulose; and/or
wherein the fabric support is prepared from natural material selected from a group comprising cotton, wool, silk and blends thereof, or synthetic material selected from a group comprising polyamide, polyester, rayon and blends thereof.

3. The composite dressing as claimed in claim 1 or 2, wherein the fabric support is pretreated with component selected from a group comprising surfactant, plasticizer and active agent or any combination thereof, followed by drying at a temperature ranging from about 20°C to about 90°C for time period ranging from about 1 hour to about 6 hours;
wherein the surfactant or the plasticizer is selected from a group comprising poloxamer, polysorbates, betaine, lecithin, lauryl sulphate,, Pluronic F-68, Sorbitantrioleate, d-α-tocopherol polyethylene glycol 1000 succinate, Polyethoxylated castor oil, polyethoxylated 12-hydroxystearic acid, glycerol, polyethylene-glycol (PEG), ethylene glycol, sorbitol, polyvinyl pyrrolidone (PVP), polyvinyl alcohol (PVA), ethylene glycol and propylene glycol or any combination thereof; and/or
wherein the active agent is selected from a group comprising pro-coagulant and natural, semisynthetic or synthetic sealant or any combination thereof, wherein the pro-coagulant is selected from a group comprising calcium chloride, calcium silicate nanoparticles, Factor X activators, prothrombin activators, fibrinogen, vitamin K, thrombin, platelet rich plasma, fibrin, tranexamic acid and aminocaproic acid or any combination thereof; wherein the sealant is surgical sealant selected from a group comprising thrombin-fibrinogen based sealant, gelatin-thrombin, albumin and thrombin solution or any combination thereof; and wherein the sealant is in liquid or semisolid form.

4. The composite dressing as claimed in any one of the preceding claims, wherein thickness of the porous matrix ranges from about 1 mm to about 100 mm, preferably from about 5 mm to 10 mm; wherein pore size of the porous matrix ranges from about 10 µm to about 300 µm; and wherein wall thickness of the porous matrix ranges from about 0.05 µm to about 5 µm; wherein thickness of the fabric support ranges from about 0.05 mm to about 5 mm; and/or wherein when embedded, thickness of interface between the layer of porous matrix and the layer of fabric support is at least about 1% of the thickness of the fabric support, preferably ranging from about 10% to about 100% of the thickness of the fabric support.

5. The composite dressing as claimed in any one of the preceding claims, wherein the fabric support is a non-woven fabric or a woven fabric; wherein the fabric substrate is composed of a special fabric obtained by mixing fibers having wetting and swelling properties different from each other; and wherein the fabric support is in a form selected from a group comprising gauze, bandage and patch or ribbon of the fabric;
wherein weight of the fabric support is at least about 1 grams per square meter (gsm); preferably ranging from about 50 gsm to about 250 gsm;
wherein the fabric support has dry breaking strength of at least about 0.1 MPa and wet breaking strength of at least about 0.01 MPa, wherein preferably the dry breaking strength ranges from about 1.4 MPa to about 3.5 MPa and the wet breaking strength ranges from about 0.5 MPa to about 2 MPa; and/or
wherein the fabric support has minimum elongation at break of at least about 5%, preferably ranging from about 50% to about 200% of the original length.

6. The composite dressing as claimed in any one of the preceding claims, wherein moisture content of the composite dressing ranges from about 5% to about 25%;
wherein elongation at break of the composite dressing ranges from about 5% to about 20% of the original length in dry conditions and about 10% to about 50% of the original length in wet conditions;
wherein tensile strength of the composite dressing ranges from about 0.5 MPa to about 10 MPa in dry conditions and about 0.1 MPa to about 5 MPa in wet conditions;
wherein the bend angle of the composite dressing is at least about 90 degrees, preferably about 180 degrees;
wherein the composite dressing has absorption capacity ranging from about 10 times to about 200 times of its original weight; and/or
wherein adhesion force of the composite dressing to application site ranges from about 0.1 MPa to 1 MPa.

7. The composite dressing as claimed in any one of the preceding claims, wherein the composite dressing contains alternating layers of the porous matrix and of the fabric support, or wherein the fabric support is in form of a string that is longer than the porous matrix, or wherein the composite dressing is in form of a tubular composite dressing with cylindrical porous matrix wrapped in tubular fabric support, or wherein the composite dressing is designed with the fabric support impregnated with a radio-opaque filament.

8. The composite dressing as claimed in claim 7, wherein when the composite dressing contains alternating layers of the porous matrix and of the fabric support, the dressing comprises 3 layers, and wherein the fabric support is sandwiched between two layers of the porous matrix or the porous matrix is sandwiched between two layers of the fabric support;
wherein when the fabric support is in form of a string that is longer than the porous matrix, the string is folded into a zig-zag fold and wherein one end of the folded fabric support is attached to the porous matrix; and
wherein when the composite dressing is designed with the fabric support impregnated with a radio-opaque filament, the radio-opaque filament is pre-attached to the fabric support or sandwiched between the fabric support and the porous matrix, and wherein the radio-opaque filament is incorporated into the composite dressing in form of a single thread, multiple threads or in a grid pattern.

9. A method for preparing the composite dressing as claimed in claim 1, wherein said method comprises:
a) contacting the fabric support with a solution of the biomaterial;
b) at least partially immersing the fabric in the solution of the biomaterial;
c) freezing the solution of the biomaterial comprising the immersed fabric support at a controlled rate; and
d) removing solvent from the frozen biomaterial solution of (c)
to obtain the composite dressing comprising the porous matrix at least partially embedded with the fabric support.

10. The method as claimed in claim 9, wherein the solution of biomaterial is prepared by dissolving the biomaterial in a solvent selected from a group comprising water, alcohol, DMSO, acid, alkali and organic solvent or any combination thereof;
wherein the acid is selected from a group comprising acetic acid, lactic acid, glycolic acid, citric acid, hydrochloric acid or any combination thereof;
wherein the alkali is selected from sodium hydroxide (NaOH), potassium hydroxide (KOH), triethanolamine and ammonia or any combination thereof;
wherein the organic solvent is selected from a group comprising dichloromethane, chloroform, acetone, ethyl acetate, methanol, isopropanol, ethanol and toluene or any combination thereof; wherein the biomaterial solution comprises pro-coagulant material or natural, semisynthetic or synthetic sealant; wherein the pro-coagulant is selected from a group comprising calcium chloride, calcium silicate nanoparticles, Factor X activators, prothrombin activators, fibrinogen, vitamin K, thrombin, platelet rich plasma, fibrin, tranexamic acid and aminocaproic acid or any combination thereof; and/or wherein the sealant is surgical sealant selected from a group comprising thrombin-fibrinogen based sealants, gelatin-thrombin, albumin and thrombin solution or any combination thereof.

11. The method as claimed in claim 10, wherein the solvent is an aqueous solution of the alcohol, DMSO, acid, alkali or organic solvent; wherein concentration of the biomaterial in the biomaterial solution ranges from about 0.1% w/v to about 10% w/v; and wherein viscosity of said biomaterial solution is maintained at atleast about 100 cP, preferably ranging from about 500 cP to about 300000 cP.

12. The method as claimed in any one of claims 9-11, wherein the fabric support is pre-treated prior to the immersion in the biomaterial; wherein the pre-treatment is by contacting the fabric support with component selected from a group comprising surfactant, plasticizer and active agent or any combination thereof, followed by drying; wherein the surfactant or the plasticizer is selected from a group comprising poloxamer, polysorbates, betaine, lecithin, lauryl sulphate, Pluronic F-68, Sorbitantrioleate, d-α-tocopherol polyethylene glycol 1000 succinate, Polyethoxylated castor oil, polyethoxylated 12-hydroxystearic acid, glycerol, polyethylene-glycol (PEG), ethylene glycol, sorbitol, polyvinyl pyrrolidone (PVP), polyvinyl alcohol (PVA), ethylene glycol and propylene glycol or any combination thereof; wherein the active agent is selected from a group comprising pro-coagulant material, natural, semisynthetic or synthetic sealants or any combinations thereof, wherein the pro-coagulant is selected from a group comprising calcium chloride, calcium silicate nanoparticles, Factor X activators, prothrombin activators, fibrinogen, vitamin K, thrombin, platelet rich plasma, fibrin, tranexamic acid and aminocaproic acid or any combination thereof; wherein the sealant is surgical sealant selected from a group comprising thrombin-fibrinogen based sealants, gelatin-thrombin, albumin and thrombin solution or any combination thereof; wherein the sealant is in liquid or semisolid form; and wherein the drying is carried out at a temperature ranging from about 20°C to about 90°C for time period ranging from about 1 hour to about 6 hours; and/or wherein the fabric support is obtained by mixing of cross-linked fibers and activated fibers having wetting and swelling properties different from each other;
wherein the cross-linked fibers are obtained by treating the biomaterial with cross-linking agent selected from a group comprising glutaraldehyde, genipin, calcium chloride, epichlorohydrin, carbodiimide derivatives, N-hydroxysuccinimide esters and combinations thereof; and wherein the activated fibers are obtained by subjecting the fibers to chemical or physical treatment selected from a group comprising acid treatment, cross-linking, exposure to water vapor, heat treatment and gamma irradiation or any combination thereof.

13. The method as claimed in any one of claims 9-12, wherein the fabric support is contacted with the solution of biomaterial such that at least about 1% of the thickness of the fabric, preferably ranging from about 10% to about 100% of thickness of the fabric support are wetted by the solution of the biomaterial; and wherein at least 50% of fibers from the fabric support remain undissolved within or outside porous matrix formed by the solution of biomaterial;
wherein the freezing is carried out at a temperature ranging from about -20°C to about 0°C for a time period ranging from about 0.5 hours to about 12 hours; and wherein rate of freezing ranges from about 0.1°C to about 5°C per minute;
wherein the solvent is removed from the frozen biomaterial solution by dipping the frozen biomaterial solution in dehydrating solvent or by carrying out freeze-drying or lyophilization; and wherein the dehydrating solvent is organic solvent selected from a group comprising alcohol, acetone and ethylacetate or any combination thereof;
wherein the frozen biomaterial solution is subjected to the lyophilization at a predefined lyophilization cycle, wherein the frozen biomaterial is exposed to a controlled vacuum of about 50 mTorr to about 250 mTorr at temperature ranging from about -40°C to about 15°C for a time period ranging from about 8 hours to about 36 hours; and secondary drying is at temperatures ranging from about 15°C to about 35°C for a time period ranging from about 2 hours to 10 hours; and/or wherein
the method further comprises saturating the composite dressing with hydration liquid selected from a group comprising water, saline, pro-coagulant material and natural, semisynthetic or synthetic sealants or any combinations thereof, followed by squeezing out excess liquid; wherein the pro-coagulant is selected from a group comprising calcium chloride, calcium silicate nanoparticles, Factor X activators, prothrombin activators, fibrinogen, vitamin K, thrombin, platelet rich plasma, fibrin, tranexamic acid and aminocaproic acid or any combination thereof; and wherein the sealant is surgical sealant selected from a group comprising thrombin-fibrinogen based sealants, gelatin-thrombin, albumin and thrombin solution or any combination thereof; and wherein the sealant is in liquid or semisolid form; and/or wherein
the method further comprises compressing the composite dressing; wherein the compressing is performed by techniques selected from a group comprising roller milling or hydraulic press.

14. A composite dressing as claimed in any one of claims 1-8, for use as a medicament.

15. The composite dressing as claimed in claim 14, wherein the medicament is used for controlling bleeding; wherein the dressing is used as a hemostatic dressing; and wherein the bleeding is caused by surgical or traumatic wounds.

16. A kit comprising the composite dressing as claimed in any one of claims 1-8 and hydration liquid, optionally along with an instruction manual; wherein the hydration liquid is selected from a group comprising water, saline, procoagulant material and natural, semisynthetic or synthetic sealants or any combinations thereof; wherein the procoagulant material is selected from a group comprising calcium chloride, calcium silicate nanoparticles, tranexamic acid, Factor X activators, Prothrombin activators, fibrin, fibrinogen, Vitamin K, thrombin, aminocaproic acid and platelet rich plasma or any combination thereof; wherein the sealant is surgical sealant selected from a group comprising thrombin-fibrinogen based sealants, gelatin-thrombin, albumin and thrombin solution or any combination thereof; and wherein the sealant is in liquid or semisolid form.

17. The kit as claimed in claim 16, for use in controlling or stopping of bleeding.

## Patentansprüche

1. Abnehmbarer Verbundverband, umfassend mindestens eine Schicht einer porösen Matrix, die aus Biomaterial zusammengesetzt ist, und mindestens eine Schicht eines Gewebeträgers, wobei der Gewebeträger mindestens teilweise in der porösen Matrix eingebettet und gelöst ist; und wobei mindestens 50% der Fasern aus dem Gewebeträger innerhalb oder außerhalb der porösen Matrix ungelöst verbleiben.

2. Verbundverband nach Anspruch 1, wobei sowohl die poröse Matrix als auch der Gewebeträger aus Biomaterial zusammengesetzt sind, und wobei sowohl die poröse Matrix als auch der Gewebeträger aus dem gleichen oder einem unterschiedlichen Biomaterial zusammengesetzt sind;
wobei das Biomaterial aus einer Gruppe ausgewählt ist, die Chitosan, Alginate, Stärke, Cellulose, Gelatine, Kollagen, Polyethylenglykol (PEG), Polyvinylalkohol (PVA), Polyurethane, Hyaluronsäure, Polylysin, Polyglutaminsäure, Poly(ethylenoxid) (PEO), Poly(propylenoxid) (PPO), Poly(acrylsäure), Poly(methacrylsäure) und deren Derivate oder jede Kombination davon umfasst;
wobei das Derivat von Chitosan aus einer Gruppe ausgewählt ist, die Chitosan-PEG und hydrophob-modifiziertes Chitosan umfasst, wobei das Derivat von Stärke aus einer Gruppe ausgewählt ist, die vorgelatinierte Stärke, Carboxymethylstärke und Natriumstärkeglykolat umfasst, und wobei das Derivat von Cellulose aus einer Gruppe ausgewählt ist, die oxidierte Cellulose, Methylcellulose und Carboxymethylcellulose umfasst; und/oder
wobei der Gewebeträger aus natürlichem Material, ausgewählt aus einer Gruppe, die Baumwolle, Wolle, Seide und Mischungen davon umfasst, oder synthetischem Material, ausgewählt aus einer Gruppe, die Polyamid, Polyester, Rayon und Mischungen davon umfasst, hergestellt ist.

3. Verbundverband nach Anspruch 1 oder 2, wobei der Gewebeträger mit einer Komponente vorbehandelt ist, die aus einer Gruppe ausgewählt ist, die ein Tensid, einen Weichmacher und einen Wirkstoff oder eine beliebige Kombination davon umfasst, gefolgt durch Trocknen bei einer Temperatur im Bereich von etwa 20°C bis etwa 90°C über einen Zeitraum von etwa 1 Stunde bis etwa 6 Stunden;
wobei das Tensid oder der Weichmacher aus einer Gruppe ausgewählt ist, die Poloxamer, Polysorbate, Betain, Lecithin, Laurylsulfat, Pluronic F-68, Sorbitantrioleat, d-α-Tocopherol-Polyethylenglykol-1000-Succinat, polyethoxyliertes Rizinusöl, polyethoxylierte 12-Hydroxystearinsäure, Glycerin, Polyethylenglykol (PEG), Ethylenglykol, Sorbit, Polyvinylpyrrolidon (PVP), Polyvinylalkohol (PVA), Ethylenglykol und Propylenglykol oder eine beliebige Kombination davon umfasst; und/oder
wobei der Wirkstoff aus einer Gruppe ausgewählt ist, die ein Prokoagulans und ein natürliches, halbsynthetisches oder synthetisches Versiegelungsmittel oder eine beliebige Kombination davon umfasst, wobei das Prokoagulans aus einer Gruppe ausgewählt ist, die Calciumchlorid, Calciumsilicat-Nanopartikel, Faktor-X-Aktivatoren, Prothrombin-Aktivatoren, Fibrinogen, Vitamin K, Thrombin, Thrombozyten-reiches Plasma, Fibrin, Tranexamsäure und Aminocapronsäure oder eine beliebige Kombination davon umfasst; wobei das Versiegelungsmittel ein chirurgisches Versiegelungsmittel ist, das aus einer Gruppe ausgewählt ist, die ein Versiegelungsmittel auf Thrombin-Fibrinogen-Basis, Gelatine-Thrombin, Albumin und Thrombinlösung oder eine beliebige Kombination davon umfasst; und wobei das Versiegelungsmittel in flüssiger oder halbfester Form vorliegt.

4. Verbundverband nach einem der vorhergehenden Ansprüche, wobei die Dicke der porösen Matrix im Bereich von etwa 1 mm bis etwa 100 mm, bevorzugt von etwa 5 mm bis 10 mm, liegt; wobei die Porengröße der porösen Matrix im Bereich von etwa 10 µm bis etwa 300 µm liegt; und wobei die Wanddicke der porösen Matrix im Bereich von etwa 0,05 µm bis etwa 5 µm liegt; wobei die Dicke des Gewebeträgers im Bereich von etwa 0,05 mm bis etwa 5 mm liegt; und/oder wobei, wenn sie eingebettet ist, die Dicke der Grenzfläche zwischen der Schicht aus poröser Matrix und der Schicht aus Gewebeträger zumindest etwa 1% der Dicke des Gewebeträgers beträgt, bevorzugt im Bereich von etwa 10% bis etwa 100% der Dicke des Gewebeträgers liegt.

5. Verbundverband nach einem der vorhergehenden Ansprüche, wobei der Gewebeträger ein Vliesstoff oder ein gewebter Stoff ist, wobei das Gewebesubstrat aus einem speziellen Stoff zusammengesetzt ist, der durch Mischen von Fasern mit voneinander unterschiedlichen Benetzungs- und Quelleigenschaften erhalten wird; und wobei der Gewebeträger in einer Form vorliegt, die aus einer Gruppe ausgewählt ist, die Verbandmull, Bandage und Pflaster oder Band aus dem Stoff umfasst;
wobei das Gewicht des Gewebeträgers zumindest etwa 1 Gramm pro Quadratmeter (gsm) beträgt und bevorzugt im Bereich von etwa 50 gsm bis etwa 250 gsm liegt;
wobei der Gewebeträger eine Trockenbruchfestigkeit von zumindest etwa 0,1 MPa und eine Nassbruchfestigkeit von zumindest etwa 0,01 MPa aufweist, wobei bevorzugt die Trockenbruchfestigkeit im Bereich von etwa 1,4 MPa bis etwa 3,5 MPa liegt und die Nassbruchfestigkeit im Bereich von etwa 0,5 MPa bis etwa 2 MPa liegt; und/oder
wobei der Gewebeträger eine Mindestbruchdehnung von zumindest etwa 5%, bevorzugt im Bereich von etwa 50% bis etwa 200% der ursprünglichen Länge, aufweist.

6. Verbundverband nach einem der vorhergehenden Ansprüche, wobei der Feuchtigkeitsgehalt des Verbundverbands im Bereich von etwa 5% bis etwa 25% liegt;
wobei die Bruchdehnung des Verbundverbands im trockenen Zustand im Bereich von etwa 5% bis etwa 20% der ursprünglichen Länge und im nassen Zustand im Bereich von etwa 10% bis etwa 50% der ursprünglichen Länge liegt;
wobei die Zugfestigkeit des Verbundverbandes im trockenen Zustand im Bereich von etwa 0,5 MPa bis etwa 10 MPa und im nassen Zustand im Bereich von etwa 0,1 MPa bis etwa 5 MPa liegt;
wobei der Biegewinkel des Verbundverbands zumindest etwa 90 Grad, bevorzugt etwa 180 Grad beträgt;
wobei der Verbundverband ein Absorptionsvermögen im Bereich des etwa 10- bis etwa 200-fachen seines ursprünglichen Gewichts aufweist; und/oder wobei die Adhäsionskraft des Verbundverbands an der Applikationsstelle im Bereich von etwa 0,1 MPa bis 1 MPa liegt.

7. Verbundverband nach einem der vorhergehenden Ansprüche, wobei der Verbundverband alternierende Schichten der porösen Matrix und des Gewebeträgers enthält, oder wobei der Gewebeträger in Form einer Schnur vorliegt, die länger ist als die poröse Matrix, oder wobei der Verbundverband in Form eines schlauchförmigen Verbundverbandes mit zylindrischer poröser Matrix vorliegt, die in einen schlauchförmigen Gewebeträger eingewickelt ist, oder wobei der Verbundverband so gestaltet ist, dass der Gewebeträger mit einem röntgendichten Filament imprägniert ist.

8. Verbundverband nach Anspruch 7, wobei, wenn der Verbundverband alternierende Schichten der porösen Matrix und des Gewebeträgers enthält, der Verband 3 Schichten umfasst, und wobei der Gewebeträger zwischen zwei Schichten der porösen Matrix oder die poröse Matrix zwischen zwei Schichten des Gewebeträgers eingebettet ist;
wobei, wenn der Gewebeträger in Form einer Schnur vorliegt, die länger als die poröse Matrix ist, die Schnur zu einer Zick-Zack-Falte gefaltet ist und wobei ein Ende des gefalteten Gewebeträgers an der porösen Matrix befestigt ist; und
wobei, wenn der Verbundverband so gestaltet ist, dass der Gewebeträger mit einem röntgendichten Filament imprägniert ist, das röntgendichte Filament an dem Gewebeträger vorfixiert ist oder zwischen dem Gewebeträger und der porösen Matrix eingebettet ist; und wobei das röntgendichte Filament in den Verbundverband in Form eines einzelnen Fadens, mehrerer Fäden oder in einem Gittermuster eingearbeitet ist.

9. Verfahren zur Herstellung des Verbundverbands nach Anspruch 1, wobei das Verfahren umfasst:
a) Inkontaktbringen des Gewebeträgers mit einer Lösung des Biomaterials,
b) zumindest teilweises Eintauchen des Gewebes in die Lösung des Biomaterials,
c) Gefrieren der Lösung des Biomaterials, die den eingetauchten Gewebeträger umfasst, bei einer kontrollierten Geschwindigkeit, und
d) Entfernen des Lösungsmittels aus der gefrorenen Biomateriallösung von (c)
um den Verbundverband zu erhalten, der die poröse Matrix umfasst, die zumindest teilweise in den Gewebeträger eingebettet ist.

10. Verfahren nach Anspruch 9, wobei die Lösung des Biomaterials durch Auflösen des Biomaterials in einem Lösungsmittel hergestellt wird, das aus einer Gruppe ausgewählt ist, die Wasser, Alkohol, DMSO, Säure, Alkali und organisches Lösungsmittel oder irgendeine Kombination davon umfasst; wobei die Säure aus einer Gruppe ausgewählt ist, die Essigsäure, Milchsäure, Glykolsäure, Zitronensäure, Salzsäure oder irgendeine Kombination davon umfasst;
wobei das Alkali ausgewählt ist aus Natriumhydroxid (NaOH), Kaliumhydroxid (KOH), Triethanolamin und Ammoniak oder einer beliebigen Kombination davon;
wobei das organische Lösungsmittel aus einer Gruppe ausgewählt ist, die Dichlormethan, Chloroform, Aceton, Ethylacetat, Methanol, Isopropanol, Ethanol und Toluol oder eine beliebige Kombination davon umfasst;
wobei die Biomateriallösung Prokoagulans oder natürliches, halbsynthetisches oder synthetisches Versiegelungsmittel umfasst, wobei das Prokoagulans aus einer Gruppe ausgewählt ist, die Calciumchlorid, Calciumsilikat-Nanopartikel, Faktor-X-Aktivatoren, Prothrombin-Aktivatoren, Fibrinogen, Vitamin K, Thrombin, Thrombozyten-reiches Plasma, Fibrin, Tranexamsäure und Aminocapronsäure oder eine beliebige Kombination davon umfasst; und/oder wobei das Versiegelungsmittel ein chirurgisches Versiegelungsmittel ist, das aus einer Gruppe ausgewählt ist, die Versiegelungsmittel auf Thrombin-Fibrinogen-Basis, Gelatine-Thrombin, Albumin und Thrombinlösung oder eine beliebige Kombination davon umfasst.

11. Verfahren nach Anspruch 10, wobei das Lösungsmittel eine wässrige Lösung eines Alkohols, DMSO, einer Säure, eines Alkalis oder eines organischen Lösungsmittels ist; wobei die Konzentration des Biomaterials in der Biomateriallösung im Bereich von etwa 0,1 Gew./Vol. bis etwa 10 Gew./Vol. liegt, und wobei die Viskosität der Biomateriallösung bei zumindest etwa 100 cP, bevorzugt im Bereich von etwa 500 cP bis etwa 300 000 cP, gehalten wird.

12. Verfahren nach einem der Ansprüche 9 bis 11, wobei der Gewebeträger vor dem Eintauchen in das Biomaterial vorbehandelt wird; wobei die Vorbehandlung durch Inkontaktbringen des Gewebeträgers mit einer Komponente erfolgt, die aus einer Gruppe ausgewählt ist, die ein Tensid, einen Weichmacher und einen Wirkstoff oder eine beliebige Kombination davon umfasst, gefolgt von Trocknen; wobei das Tensid oder der Weichmacher aus einer Gruppe ausgewählt ist, die Poloxamer, Polysorbate, Betain, Lecithin, Laurylsulfat, Pluronic F-68, Sorbitantrioleat, d-α-Tocopherol-Polyethylenglykol-1000-Succinat, polyethoxyliertes Rizinusöl, polyethoxylierte 12-Hydroxystearinsäure, Glycerin, Polyethylenglykol (PEG), Ethylenglykol, Sorbitol, Polyvinylpyrrolidon (PVP), Polyvinylalkohol (PVA), Ethylenglykol und Propylenglykol oder eine beliebige Kombination davon umfasst; wobei der Wirkstoff aus einer Gruppe ausgewählt ist, die Prokoagulans, natürliche, halbsynthetische oder synthetische Versiegelungsmittel oder beliebige Kombinationen davon umfasst, wobei das Prokoagulans aus einer Gruppe ausgewählt ist, die Calciumchlorid, Calciumsilikat-Nanopartikel, Faktor-X-Aktivatoren, Prothrombin-Aktivatoren, Fibrinogen, Vitamin K, Thrombin, Thrombozyten-reiches Plasma, Fibrin, Tranexamsäure und Aminocapronsäure oder eine beliebige Kombination davon umfasst; wobei das Versiegelungsmittel ein chirurgisches Versiegelungsmittel ist, das aus einer Gruppe ausgewählt ist, die Versiegelungsmittel auf Thrombin-Fibrinogen-Basis, Gelatine-Thrombin, Albumin und Thrombinlösung oder eine beliebige Kombination davon umfasst; wobei das Versiegelungsmittel in flüssiger oder halbfester Form vorliegt; und wobei das Trocknen bei einer Temperatur im Bereich von etwa 20°C bis etwa 90°C über einen Zeitraum von etwa 1 Stunde bis etwa 6 Stunden durchgeführt wird, und/oder
wobei der Gewebeträger durch Mischen von vernetzten Fasern und aktivierten Fasern mit voneinander unterschiedlichen Benetzungs- und Quelleigenschaften erhalten wird,
wobei die vernetzten Fasern durch Behandlung des Biomaterials mit einem Vernetzungsmittel erhalten werden, das aus einer Gruppe ausgewählt ist, die Glutaraldehyd, Genipin, Calciumchlorid, Epichlorhydrin, Carbodiimid-Derivate, N-Hydroxysuccinimidestern und Kombinationen davon umfasst; wobei die aktivierten Fasern durch eine chemische oder physikalische Behandlung erhalten werden, die aus einer Gruppe ausgewählt ist, die eine Säurebehandlung, Vernetzung, Wasserdampfeinwirkung, Wärmebehandlung und Gammabestrahlung oder eine Kombination davon umfasst.

13. Verfahren nach einem der Ansprüche 9 bis 12, wobei der Gewebeträger mit der Lösung des Biomaterials so in Kontakt gebracht wird, dass mindestens etwa 1% der Dicke des Gewebes, bevorzugt im Bereich von etwa 10% bis etwa 100% der Dicke des Gewebeträgers, von der Lösung des Biomaterials benetzt werden; und wobei mindestens 50% der Fasern aus dem Gewebeträger innerhalb oder außerhalb der durch die Lösung des Biomaterials gebildeten porösen Matrix ungelöst bleiben;
wobei das Gefrieren bei einer Temperatur im Bereich von etwa -20°C bis etwa 0°C für eine Zeitdauer im Bereich von etwa 0,5 Stunden bis etwa 12 Stunden durchgeführt wird; und wobei die Gefriergeschwindigkeit im Bereich von etwa 0,1°C bis etwa 5°C pro Minute liegt;
wobei das Lösungsmittel aus der gefrorenen Biomateriallösung durch Eintauchen der gefrorenen Biomateriallösung in Entwässerungslösungsmittel oder durch Durchführen von Gefriertrocknung oder Lyophilisierung entfernt wird; und wobei das Entwässerungslösungsmittel ein organisches Lösungsmittel ist, das aus einer Gruppe ausgewählt ist, die Alkohol, Aceton und Ethylacetat oder eine beliebige Kombination davon umfasst;
wobei die gefrorene Biomateriallösung der Gefriertrocknung bei einem vordefinierten Gefriertrocknungszyklus unterzogen wird, wobei das gefrorene Biomaterial einem kontrollierten Vakuum von etwa 50 mTorr bis etwa 250 mTorr bei einer Temperatur im Bereich von etwa -40°C bis etwa 15°C für eine Zeitdauer von etwa 8 Stunden bis etwa 36 Stunden ausgesetzt wird; und Sekundärtrocknung bei Temperaturen im Bereich von etwa 15°C bis etwa 35°C für eine Zeitdauer im Bereich von etwa 2 Stunden bis 10 Stunden erfolgt; und/oder
das Verfahren ferner das Sättigen des Verbundverbands mit einer Hydratationsflüssigkeit umfasst, die aus einer Gruppe ausgewählt ist, die Wasser, Kochsalzlösung, Prokoagulans und natürliche, halbsynthetische oder synthetische Versiegelungsmittel oder beliebige Kombinationen davon umfasst, gefolgt von Herauspressen überschüssiger Flüssigkeit; wobei das Prokoagulans aus einer Gruppe ausgewählt ist, die Calciumchlorid, Calciumsilikat-Nanopartikel, Faktor-X-Aktivatoren, Prothrombin-Aktivatoren, Fibrinogen, Vitamin K, Thrombin, Thrombozyten-reiches Plasma, Fibrin, Tranexamsäure und Aminocapronsäure oder eine beliebige Kombination davon umfasst; und wobei das Versiegelungsmittel ein chirurgisches Versiegelungsmittel ist, das aus einer Gruppe ausgewählt ist, die Versiegelungsmittel auf Thrombin-Fibrinogen-Basis, Gelatine-Thrombin, Albumin und Thrombinlösung oder eine beliebige Kombination davon umfasst; und wobei das Versiegelungsmittel in flüssiger oder halbfester Form vorliegt; und/oder wobei
das Verfahren ferner das Komprimieren des Verbundverbands umfasst, wobei das Komprimieren durch Techniken durchgeführt wird, die aus einer Gruppe ausgewählt sind, die Rollwalzen oder hydraulische Pressen umfasst.

14. Verbundverband nach einem der Ansprüche 1 bis 8 zur Verwendung als Medikament.

15. Verbundverband nach Anspruch 14, wobei das Medikament zur Kontrolle von Blutungen verwendet wird; wobei der Verband als hämostatischer Verband verwendet wird; und wobei die Blutung durch chirurgische oder traumatische Wunden verursacht wird.

16. Kit, umfassend den Verbundverband nach einem der Ansprüche 1 bis 8 und eine Hydratationsflüssigkeit, optional zusammen mit einer Gebrauchsanweisung; wobei die Hydratationsflüssigkeit aus einer Gruppe ausgewählt ist, die Wasser, Kochsalzlösung, Prokoagulans und natürliche, halbsynthetische oder synthetische Versiegelungsmittel oder beliebige Kombinationen davon umfasst; wobei das Prokoagulans aus einer Gruppe ausgewählt ist, die Calciumchlorid, Calciumsilikat-Nanopartikel, Tranexamsäure, Faktor X-Aktivatoren, Prothrombin-Aktivatoren, Fibrin, Fibrinogen, Vitamin K, Thrombin, Aminocapronsäure und Thrombozyten-reiches Plasma oder eine beliebige Kombination davon umfasst; wobei das Versiegelungsmittel ein chirurgisches Versiegelungsmittel ist, das aus einer Gruppe ausgewählt ist, die Versiegelungsmittel auf Thrombin-Ffibrinogen-Basis, Gelatine-Thrombin, Albumin und Thrombinlösung oder eine beliebige Kombination davon umfasst; und wobei das Versiegelungsmittel in flüssiger oder halbfester Form vorliegt.

17. Kit nach Anspruch 16 zur Verwendung bei der Kontrolle oder Stillung von Blutungen.

## Revendications

1. - Pansement composite amovible comprenant au moins une couche de matrice poreuse composée de biomatériau et au moins une couche de support en tissu, le support en tissu étant au moins partiellement incorporé et dissous dans la matrice poreuse, et au moins 50 % de fibres provenant du support en tissu restant non dissoutes à l'intérieur ou à l'extérieur de la matrice poreuse.

2. - Pansement composite selon la revendication 1, dans lequel la matrice poreuse et le support en tissu sont tous deux composés de biomatériau, et dans lequel la matrice poreuse et le support en tissu sont tous deux composés de biomatériaux identiques ou différents;
dans lequel le biomatériau est choisi dans un groupe comprenant le chitosane, les alginates, l'amidon, la cellulose, la gélatine, le collagène, le polyéthylène glycol (PEG), l'alcool polyvinylique (PVA), les polyuréthanes, l'acide hyaluronique, la poly-lysine, l'acide poly-glutamique, le poly(oxyde d'éthylène) (PEO), le poly(oxyde de propylène) (PPO), l'acide polyacrylique, l'acide polyméthacrylique et leurs dérivés ou toute combinaison de ceux-ci;
dans lequel le dérivé de chitosane est choisi dans un groupe comprenant le chitosane-PEG et le chitosane modifié de manière hydrophobe; dans lequel le dérivé d'amidon est choisi dans un groupe comprenant l'amidon prégélatinisé, le carboxyméthyl amidon et le glycolate d'amidon sodique; et dans lequel le dérivé de cellulose est choisi dans un groupe comprenant la cellulose oxydée, la méthyl cellulose et la carboxyméthyl cellulose; et/ou
dans lequel le support en tissu est préparé à partir d'une matière naturelle choisie dans un groupe comprenant le coton, la laine, la soie et les mélanges de ceux-ci, ou d'une matière synthétique choisie dans un groupe comprenant le polyamide, le polyester, la rayonne et les mélanges de ceux-ci.

3. - Pansement composite selon l'une des revendications 1 ou 2, dans lequel le support en tissu est prétraité par un composant choisi dans un groupe comprenant un tensio-actif, un plastifiant et un agent actif ou toute combinaison de ceux-ci, en faisant suivre par un séchage à une température se situant dans la plage d'environ 20°C à environ 90°C pendant une période se situant dans la plage d'environ 1 heure à environ 6 heures;
dans lequel le tensio-actif ou le plastifiant est choisi dans un groupe comprenant un poloxamère, les polysorbates, la bétaïne, la lécithine, un lauryl sulfate, le Pluronic F-68, le trioléate de sorbitane, le d-α-tocophérol polyéthylène glycol 1000 succinate, l'huile de ricin polyéthoxylée, l'acide 12-hydroxystéarique polyéthoxylé, le glycérol, le polyéthylène-glycol (PEG), l'éthylène glycol, le sorbitol, la polyvinyl pyrrolidone (PVP), l'alcool polyvinylique (PVA), l'éthylène glycol et le propylène glycol ou toute combinaison de ceux-ci; et/ou
dans lequel l'agent actif est choisi dans un groupe comprenant un pro-coagulant et un agent d'étanchéité naturel, semi-synthétique ou synthétique ou toute combinaison de ceux-ci, dans lequel le pro-coagulant est choisi dans un groupe comprenant le chlorure de calcium, les nanoparticules de silicate de calcium, les activateurs du Facteur X, les activateurs de la prothrombine, le fibrinogène, la vitamine K, la thrombine, le plasma riche en plaquettes, la fibrine, l'acide tranexamique et l'acide aminocaproïque ou toute combinaison de ceux-ci; dans lequel l'agent d'étanchéité est un agent d'étanchéité chirurgical choisi dans un groupe comprenant un agent d'étanchéité à base de thrombine - fibrinogène, la gélatine-thrombine, une solution d'albumine et de thrombine ou toute combinaison de ceux-ci; et dans lequel l'agent d'étanchéité est sous forme liquide ou semi-solide.

4. - Pansement composite selon l'une quelconque des revendications précédentes, dans lequel une épaisseur de la matrice poreuse se situe dans la plage d'environ 1 mm à environ 100 mm, de préférence d'environ 5 mm à 10 mm; dans lequel la taille de pore de la matrice poreuse se situe dans la plage d'environ 10 µm à environ 300 µm; et dans lequel l'épaisseur de paroi de la matrice poreuse se situe dans la plage d'environ 0,05 µm à environ 5 µm; dans lequel une épaisseur du support en tissu se situe dans la plage d'environ 0,05 mm à environ 5 mm; et/ou dans lequel, lorsqu'elle est incorporée, une épaisseur d'interface entre la couche de matrice poreuse et la couche de support en tissu est d'au moins environ 1% de l'épaisseur du support en tissu, de préférence se situant dans la plage d'environ 10% à environ 100% de l'épaisseur du support en tissu.

5. - Pansement composite selon l'une quelconque des revendications précédentes, dans lequel le support en tissu est un tissu non tissé ou un tissu tissé; dans lequel le substrat en tissu est composé d'un tissu spécial obtenu par mélange de fibres ayant des propriétés de mouillage et de gonflement différentes les unes des autres; et dans lequel le support en tissu est sous une forme choisie dans un groupe comprenant une gaze, un bandage et une pièce ou un ruban du tissu;
dans lequel un poids du support en tissu est d'au moins environ 1 gramme par mètre carré (g/m²), de préférence se situant dans la plage d'environ 50 g/m² à environ 250 g/m²;
dans lequel le support en tissu a une résistance à la rupture à sec d'au moins environ 0,1 MPa et une résistance à la rupture à l'état humide d'au moins environ 0,01 MPa, dans lequel, de préférence, la résistance à la rupture à sec se situe dans la plage d'environ 1,4 MPa à environ 3,5 MPa et la résistance à la rupture à l'état humide se situe dans la plage d'environ 0,5 MPa à environ 2 MPa; et/ou
dans lequel le support en tissu a un allongement minimal à la rupture d'au moins environ 5 %, de préférence se situant dans la plage d'environ 50 % à environ 200 % de la longueur initiale.

6. - Pansement composite selon l'une quelconque des revendications précédentes, dans lequel une teneur en humidité du pansement composite se situe dans la plage d'environ 5 % à environ 25 %;
dans lequel l'allongement à la rupture du pansement composite se situe dans la plage d'environ 5 % à environ 20 % de la longueur initiale dans des conditions sèches et d'environ 10 % à environ 50 % de la longueur initiale dans des conditions humides;
dans lequel la résistance à la traction du pansement composite se situe dans la plage d'environ 0,5 MPa à environ 10 MPa dans des conditions sèches et d'environ 0,1 MPa à environ 5 MPa dans des conditions humides;
dans lequel l'angle de cintrage du pansement composite est d'au moins environ 90 degrés, de préférence d'environ 180 degrés;
dans lequel le pansement composite a une capacité d'absorption se situant dans la plage d'environ 10 fois à environ 200 fois son poids initial; et/ou
dans lequel la force d'adhérence du pansement composite au site d'application se situe dans la plage d'environ 0,1 MPa à 1 MPa.

7. - Pansement composite selon l'une quelconque des revendications précédentes, dans lequel le pansement composite contient des couches alternées de la matrice poreuse et du support en tissu, ou dans lequel le support en tissu est sous la forme d'un cordon qui est plus long que la matrice poreuse, ou dans lequel le pansement composite est sous la forme d'un pansement composite tubulaire avec une matrice poreuse cylindrique enveloppée dans un support en tissu tubulaire, ou dans lequel le pansement composite est conçu avec le support en tissu imprégné d'un filament radio-opaque.

8. - Pansement composite selon la revendication 7, dans lequel, lorsque le pansement composite contient des couches alternées de la matrice poreuse et du support en tissu, le pansement comprend 3 couches, et dans lequel le support en tissu est pris en sandwich entre deux couches de la matrice poreuse ou la matrice poreuse est prise en sandwich entre deux couches du support en tissu;
dans lequel, lorsque le support en tissu est sous la forme d'un cordon qui est plus long que la matrice poreuse, le cordon est plié en un pli en zig-zag et dans lequel une extrémité du support en tissu plié est fixée à la matrice poreuse; et
dans lequel, lorsque le pansement composite est conçu avec le support en tissu imprégné d'un filament radio-opaque, le filament radio-opaque est pré-attaché au support en tissu ou pris en sandwich entre le support en tissu et la matrice poreuse, et dans lequel le filament radio-opaque est incorporé dans le pansement composite sous la forme d'un fil unique, de multiples fils ou dans un motif de grille.

9. - Procédé de fabrication du pansement composite selon la revendication 1, dans lequel ledit procédé comprend:
a) mettre en contact le support en tissu avec une solution du biomatériau;
b) immerger au moins partiellement le tissu dans la solution du biomatériau;
c) congeler la solution du biomatériau comprenant le support en tissu immergé à une vitesse contrôlée; et
d) retirer le solvant de la solution de biomatériau congelée de (c)
pour obtenir le pansement composite comprenant la matrice poreuse au moins partiellement intégrée avec le support en tissu.

10. - Procédé selon la revendication 9, dans lequel la solution de biomatériau est préparée par dissolution du biomatériau dans un solvant choisi dans un groupe comprenant l'eau, un alcool, le DMSO, un acide, un alcali et un solvant organique ou toute combinaison de ceux-ci;
dans lequel l'acide est choisi dans un groupe comprenant l'acide acétique, l'acide lactique, l'acide glycolique, l'acide citrique, l'acide chlorhydrique ou toute combinaison de ceux-ci;
dans lequel l'alcali est choisi parmi l'hydroxyde de sodium (NaOH), l'hydroxyde de potassium (KOH), la triéthanolamine et l'ammoniaque ou toute combinaison de ceux-ci;
dans lequel le solvant organique est choisi dans un groupe comprenant le dichlorométhane, le chloroforme, l'acétone, l'acétate d'éthyle, le méthanol, l'isopropanol, l'éthanol et le toluène ou toute combinaison de ceux-ci;
dans lequel la solution de biomatériau comprend un matériau pro-coagulant ou un agent d'étanchéité naturel, semi-synthétique ou synthétique; dans lequel le pro-coagulant est choisi dans un groupe comprenant le chlorure de calcium, les nanoparticules de silicate de calcium, les activateurs du Facteur X, les activateurs de la prothrombine, le fibrinogène, la vitamine K, la thrombine, le plasma riche en plaquettes, la fibrine, l'acide tranexamique et l'acide aminocaproïque ou toute combinaison de ceux-ci; et/ou dans lequel l'agent d'étanchéité est un agent d'étanchéité chirurgical choisi dans un groupe comprenant les agents d'étanchéité à base de thrombine-fibrinogène, la gélatine-thrombine, une solution d'albumine et de thrombine ou toute combinaison de ceux-ci.

11. - Procédé selon la revendication 10, dans lequel le solvant est une solution aqueuse de l'alcool, du DMSO, d'un acide, d'un alcali ou d'un solvant organique; dans lequel la concentration du biomatériau dans la solution de biomatériau se situe dans la plage d'environ 0,1 % p/v à environ 10 % p/v; et dans lequel une viscosité de ladite solution de biomatériau est maintenue à au moins environ 100 cP, de préférence se situant dans la plage d'environ 500 cP à environ 300 000 cP.

12. - Procédé selon l'une quelconque des revendications 9 à 11, dans lequel le support en tissu est prétraité avant l'immersion dans le biomatériau; dans lequel le prétraitement s'effectue par mise en contact du support en tissu avec un composant choisi dans un groupe comprenant un tensio-actif, un plastifiant et un agent actif ou toute combinaison de ceux-ci, en faisant suivre par un séchage; dans lequel le tensio-actif ou le plastifiant est choisi dans un groupe comprenant un poloxamère, les polysorbates, la bétaïne, la lécithine, un lauryl sulfate, le Pluronic F-68, le trioléate de sorbitane, le d-α-tocophérol polyéthylène glycol 1000 succinate, l'huile de ricin polyéthoxylée, l'acide 12-hydroxystéarique polyéthoxylé, le glycérol, le polyéthylène-glycol (PEG), l'éthylène glycol, le sorbitol, la polyvinyl pyrrolidone (PVP), l'alcool polyvinylique (PVA), l'éthylène glycol et le propylène glycol ou toute combinaison de ceux-ci; dans lequel l'agent actif est choisi dans un groupe comprenant un matériau pro-coagulant, des agents d'étanchéité naturels, semi-synthétiques ou synthétiques ou toute combinaison de ceux-ci, dans lequel le pro-coagulant est choisi dans un groupe comprenant le chlorure de calcium, les nanoparticules de silicate de calcium, les activateurs du Facteur X, les activateurs de la prothrombine, le fibrinogène, la vitamine K, la thrombine, le plasma riche en plaquettes, la fibrine, l'acide tranexamique et l'acide aminocaproïque ou toute combinaison de ceux-ci; dans lequel l'agent d'étanchéité est un agent d'étanchéité chirurgical choisi dans un groupe comprenant les agents d'étanchéité à base de thrombine-fibrinogène, la gélatine-thrombine, une solution d'albumine et de thrombine ou toute combinaison de ceux-ci; dans lequel l'agent d'étanchéité est sous forme liquide ou semi-solide; et dans lequel le séchage est effectué à une température se situant dans la plage d'environ 20°C à environ 90°C pendant une période de temps se situant dans la plage d'environ 1 heure à environ 6 heures; et/ou
dans lequel le support en tissu est obtenu par mélange de fibres réticulées et de fibres activées ayant des propriétés de mouillage et de gonflement différentes les unes des autres;
dans lequel les fibres réticulées sont obtenues par traitement du biomatériau par un agent de réticulation choisi dans un groupe comprenant le glutaraldéhyde, la génipine, le chlorure de calcium, l'épichlorhydrine, les dérivés de carbodimide, les esters de N-hydroxysuccinimide et les combinaisons de ceux-ci; et dans lequel les fibres activées sont obtenues en soumettant les fibres à un traitement chimique ou physique choisi dans un groupe comprenant un traitement par acide, une réticulation, une exposition à la vapeur d'eau, un traitement thermique et une irradiation gamma ou toute combinaison de ceux-ci.

13. - Procédé selon l'une quelconque des revendications 9 à 12, dans lequel le support en tissu est mis en contact avec la solution de biomatériau de telle sorte qu'au moins environ 1 % de l'épaisseur du tissu, de préférence d'environ 10 % à environ 100 % de l'épaisseur du support en tissu, est mouillé par la solution du biomatériau; et dans lequel au moins 50 % des fibres provenant du support en tissu restent non dissoutes à l'intérieur ou à l'extérieur de la matrice poreuse formée par la solution de biomatériau;
dans lequel la congélation est effectuée à une température se situant dans la plage d'environ -20°C à environ 0°C pendant une période de temps se situant dans la plage d'environ 0,5 heure à environ 12 heures; et dans lequel une vitesse de congélation se situe dans la plage d'environ 0,1°C et environ 5°C par minute;
dans lequel le solvant est retiré de la solution de biomatériau congelée par immersion de la solution de biomatériau congelée dans un solvant déshydratant ou en effectuant une cryodessiccation ou une lyophilisation; et dans lequel le solvant déshydratant est un solvant organique choisi dans un groupe comprenant un alcool, l'acétone et l'acétate d'éthyle ou toute combinaison de ceux-ci;
dans lequel la solution de biomatériau congelée est soumise à la lyophilisation à un cycle de lyophilisation prédéfini, dans lequel le biomatériau congelé est exposé à un vide contrôlé d'environ 50 mTorr à environ 250 mTorr à une température se situant dans la plage d'environ -40°C à environ 15°C pendant une période de temps se situant dans la plage d'environ 8 heures à environ 36 heures; et un séchage secondaire se fait à des températures se situant dans la plage d'environ 15°C à environ 35°C pendant une période de temps se situant dans la plage d'environ 2 heures à 10 heures; et/ou
dans lequel le procédé comprend en outre une saturation du pansement composite avec un liquide d'hydratation choisi dans un groupe comprenant de l'eau, une solution saline, un matériau pro-coagulant et des agents d'étanchéité naturels, semi-synthétiques ou synthétiques ou toute combinaison de ceux-ci, en faisant suivre par un essorage de liquide en excès; dans lequel le pro-coagulant est choisi dans un groupe comprenant le chlorure de calcium, les nanoparticules de silicate de calcium, les activateurs du Facteur X, les activateurs de prothrombine, le fibrinogène, la vitamine K, la thrombine, le plasma riche en plaquettes, la fibrine, l'acide tranexamique et l'acide aminocaproïque ou toute combinaison de ceux-ci; et dans lequel l'agent d'étanchéité est un agent d'étanchéité chirurgical choisi dans un groupe comprenant les agents d'étanchéité à base de thrombine-fibrinogène, la gélatine-thrombine, une solution d'albumine et de thrombine ou toute combinaison de ceux-ci; et dans lequel l'agent d'étanchéité est sous forme liquide ou semi-solide; et/ou
dans lequel le procédé comprend en outre une compression du pansement composite; dans lequel la compression est réalisée par des techniques choisies dans un groupe comprenant un laminage par rouleaux ou une presse hydraulique.

14. - Pansement composite selon l'une quelconque des revendications 1 à 8, pour une utilisation comme médicament.

15. - Pansement composite selon la revendication 14, dans lequel le médicament est utilisé pour contrôler un saignement; dans lequel le pansement est utilisé comme pansement hémostatique; et dans lequel le saignement est causé par des blessures chirurgicales ou traumatiques.

16. - Kit comprenant le pansement composite selon l'une quelconque des revendications 1 à 8 et un liquide d'hydratation, facultativement conjointement avec un manuel d'instructions; dans lequel le liquide d'hydratation est choisi dans un groupe comprenant l'eau, une solution saline, un matériau pro-coagulant et les agents d'étanchéité naturels, semi-synthétiques ou synthétiques ou toute combinaison de ceux-ci; dans lequel le matériau pro-coagulant est choisi dans un groupe comprenant le chlorure de calcium, les nanoparticules de silicate de calcium, l'acide tranexamique, les activateurs du Facteur X, les activateurs de la prothrombine, la fibrine, le fibrinogène, la vitamine K, la thrombine, l'acide aminocaproïque et le plasma riche en plaquettes ou toute combinaison de ceux-ci; dans lequel l'agent d'étanchéité est un agent d'étanchéité chirurgical choisi dans un groupe comprenant les agents d'étanchéité à base de thrombine-fibrinogène, la gélatine-thrombine, une solution d'albumine et de thrombine ou toute combinaison de ceux-ci; et dans lequel l'agent d'étanchéité est sous forme liquide ou semi-solide.

17. - Kit selon la revendication 16, pour une utilisation dans le contrôle ou l'arrêt d'un saignement.
